# EUROPEAN PATENT SPECIFICATION

(11) **EP 3 193 856 B1**
(45) Date of publication and mention of the grant of the patent: **11.08.2021**
(21) Application number: 15766490.5
(22) Date of filing: 18.09.2015
(51) Int. Cl.: A61K 31/17, A61K 31/33, A61P 33/00

(54) **FORMULATION**
FORMULIERUNG
FORMULATION

(30) Priority: 19.09.2014 GB 201416633; 26.06.2015 GB 201511301
(43) Date of publication of application: 26.07.2017
(73) Proprietor: Pharmaq AS, 7863 Overhalla (NO)
(72) Inventor: BREKKE, Stian, N-0213 Oslo (NO); KARLSEN, Marius, N-0213 Oslo (NO); GRONGSTAD, Mari Skurdal, N-0213 Oslo (NO); ANDERSEN, Rachmilla Souhoka, N-0213 Oslo (NO); LOKLING, Knut-Egil, N-0213 Oslo (NO); AKSNES, Elin, N-0213 Oslo (NO)
(74) Representative: Mannion, Sally Kim
(86) International application number: PCT/EP2015/071505
(87) International publication number: WO 2016/042154

(56) References cited:
- WO-A1-2014/016252
- FR-A1- 2 720 898
- None

## Description

### Field of the Invention

In the broadest aspect, the present invention relates to the field of controlling diseases in fish. More specifically, the invention relates to injectable formulations for controlling parasitic infestations, such as infestations with sea lice.

### Background

Parasitic infestations constitute considerable problems in the fish farming industry. Infestation with sea lice in particular (such as *Lepeophtheirus salmonis, Caligus elongatus,* and *Caligus rogercresseyi*) is considered to be one of the most important disease problems in the farming of salmonids, especially in Atlantic salmon (*Salmo salar*) and rainbow trout (*Oncorhynchus mykiss*).

Developing resistance by parasites against currently available products presents a major problem for the fish industry. Parasite control in commercial fish farming largely involves bath treatment with substances such as organophosphates, pyrethroids and hydrogen peroxide. Alternatively, substances such as chitin synthesis inhibitors or macrocyclic lactones may be administered orally, for example mixed with feed, and can be effective against parasitic diseases in fish.

Treatments may also be administered to fish by means of intraperitoneal injection, however this requires extensive handling of the fish, causing stress and potentially mortality since the fish must be pumped between holding tanks and tanks containing anesthetic. Due to the large scale of modern aquaculture such treatment programs are labour-intensive and expensive. Aqueous preparations of chitin synthesis inhibitors suitable for intraperitoneal injection have nevertheless previously been described. WO 99/063824 discloses aqueous preparations of inhibitors including hexaflumuron, however the formulation provides only a relatively low bioavailability. WO 2014/016252 discloses an injectable formulation comprising an aqueous suspension of nanoparticulate hexaflumuron or lufenuron.

There is a need for an improved formulation for the treatment of fish parasite infestations, wherein the formulations deliver a high bioavailability, are physiochemically stable over time, and can be administered in an industrially convenient manner.

It has now surprisingly been found that non-aqueous injectable formulations may be produced, comprising a parasiticide in homogeneous non-aqueous solution. Moreover, these formulations are antiparasitically effective, non-toxic to the fish, demonstrate good inhibitor stability and bioavailability, and provide robust protection against parasites for an extended period of time after administration.

### Summary

In accordance with a first aspect of the present invention, there is provided a parasiticide formulation. The formulation is not toxic to fish when administered by intraperitoneal injection. The formulation is a homogeneous non-aqueous parasiticide solution, and the formulation comprises:
(a) a hydrophilic polymer; and/or,
(b) a non-aqueous polar solvent, present in an amount of 5-40% by weight.

In some embodiments, the formulation comprises a hydrophilic polymer.

In some embodiments, the formulation comprises a hydrophilic polymer and a non-aqueous polar solvent, present in an amount of 5-40% by weight.

In some embodiments, the formulation comprises a hydrophilic polymer, a non-aqueous polar solvent, present in an amount of 5-40% by weight, and a solubilizer.

In some embodiments, the formulation comprises a non-aqueous polar solvent, present in an amount of 5-40% by weight, and a solubilizer.

When administered by intraperitoneal injection to Atlantic salmon (24-26 grams) at a water temperature of about 17 degrees C, the formulation induces:
(a) no increased mortality when administered in an amount of 0.05ml; and,
(b) at most a 40% increase in mortality when administered in an amount of 0.2ml. Increased mortality is determined after 7 days relative to a control-treated group, in other words, relative to an identical fish population to which a control formulation, such as PBS, is administered by intraperitoneal injection in an amount of 0.05ml, or 0.2ml as appropriate.

In some embodiments, the formulation is not toxic to salmonids when administered by intraperitoneal injection.

In some embodiments, the formulation is suitable for treating sea lice by intraperitoneal injection of host fish.

No water is added during the production of the formulation. As the skilled person will appreciate, however, in some circumstances a small amount of water may be present in the formulation, due, for example, to hygroscopic properties of one or more of the constituent agents. Thus, the formulation is a non-aqueous solution and contains at most 4%, 3%, or 2%, and preferably at most 1.5%, 1% or 0.5% water. The formulation is homogeneous and consists of a single uniform non-aqueous phase. The formulation does not comprise a suspension, a dispersion, or an emulsion, and is a non-micellar or substantially non-micellar solution.

The parasiticide may be insoluble in water. The parasiticide may have a solubility in water of less than 0.1 mg/ml. The parasiticide may comprise a chitin synthesis inhibitor. The chitin synthesis inhibitor may consist of or comprise one or a combination of hexaflumuron, diflubenzuron, lufenuron, deltamethrin, ivermectin, or emamectin. Preferably the parasiticide is one or a combination of hexaflumuron, diflubenzuron, or lufenuron, and most preferably the parasiticide comprises hexaflumuron.

In some embodiments, the formulation comprises a parasiticide in solution in a hydrophilic polymer. In these embodiments, the parasiticide may be present in the formulation in an amount of 10-150 mg/ml, such as 15-120, 20-100, 90-100, 25-90 or 30-80 mg/ml.

In some embodiments, the formulation comprises a parasiticide in solution in a combination of a hydrophilic polymer, such as PEG400, and a non-aqueous polar solvent, such as DMSO. The non-aqueous polar solvent may be present in the formulation in an amount of 5-40% by weight, such as about 15% by weight. In these embodiments, the parasiticide may be present in the formulation in an amount of 10-300 mg/ml, such as 50-260, 60-200, 70-180, 75-140, 80-130, 85-120, or 90-100 mg/ml. Preferably, the parasiticide is present in the formulation in an amount of about 90 mg/ml.

In embodiments in which the formulation comprises a parasiticide in solution in a hydrophilic polymer, the formulation may further comprise a solubilizer.

In some embodiments, the formulation comprises a parasiticide in solution in a non-aqueous polar solvent. The non-aqueous polar solvent is present in an amount of 5-40% by weight, and the formulation further comprises a solubilizer. In these embodiments, the parasiticide may be present in the formulation in an amount of 10-300 mg/ml, such as 50-260, 60-230, 70-190, 75-170, 80-150, 85-120, or 90-100 mg/ml. When the non-aqueous polar solvent is present in the formulation in a lower amount, such as an amount of less than 35%, less than 30%, less than 25%, such as, for example or 10-20%, the parasiticide may be present in an amount of 10-300 mg/ml, such as 50-230, 70-190, 75-170, 80-150, 85-120, most preferably 90-100 mg/ml.

In some embodiments, the formulation comprises a parasiticide in solution in a non-aqueous polar solvent. The non-aqueous polar solvent is present in an amount of 5-40% by weight, and the formulation further comprises a solubilizer and a hydrophilic polymer. In these embodiments, the parasiticide is present in an amount of 10-300 mg/ml, such as 50-260, 60-230, 70-190, 75-170, 80-150, 85-120, or 90-100 mg/ml.

When the formulation comprises a hydrophilic polymer, the hydrophilic polymer may have a molecular weight in the range of 50-2000, such as 100-1800. The hydrophilic polymer may consist of or comprise one or a combination of PEG, a PEG derivative, PPG, or a PPG derivative. Preferably, the hydrophilic polymer comprises a monofunctional PEG, a homobifunctional PEG, a heterobifunctional PEG, a multi-arm PEG, a star-PEG, a polyether, a homobifunctional PPG, or a monofunctional PPG. More preferably the hydrophilic polymer comprises PEG, and the hydrophilic polymer may be PEG having a molecular weight of between 200 and 1000 g/mol. Preferably the hydrophilic polymer comprises PEG300, PEG400, or PEG600. The hydrophilic polymer may be present in the formulation in an amount of 5-100% by weight, such as 10-99%, 15-98%, 20-97%, 25-96%, 30-95%, 35-94%, 40-93%, 45-92%, 50-91%, 55-90%, 60-89%, 65-88%, 70-87%, 75-86%, 80-85%. Preferably, the hydrophilic polymer is present in the formulation in an amount of about 70-90%, most preferably about 85% by weight.

When the formulation comprises a combination of one or more hydrophilic polymer(s) and one or more non-aqueous polar solvent(s), the hydrophilic polymer(s) may be present in a total amount of 5-97%, such as 25-95%, or 50-90%, such as 75-87% by weight. In these embodiments, the non-aqueous polar solvent(s) may be present in the formulation in a total amount of 5-35% by weight, such as 7-20% or 10-15% by weight. Preferably, when the formulation comprises a combination of one or more hydrophilic polymer(s) and one or more non-aqueous polar solvent(s), the hydrophilic polymer is present in the formulation in a total amount of about 85% by weight, and the non-aqueous polar solvent is present in the formulation in a total amount of about 15% by weight.

When the formulation comprises a non-aqueous polar solvent, the non-aqueous polar solvent may consist of or comprise one or a combination of DMSO, NMP, Tetraglycol, acetone, or DMF. Preferably the non-aqueous polar solvent comprises DMSO. The non-aqueous polar solvent may be present in the formulation in an amount of 5-35% by weight, and preferably may be present in an amount of 5-30%, 7-25%, 10-20%, or 12-15% by weight. Preferably, the non-aqueous polar solvent is present in the formulation in an amount of about 15% by weight.

When the formulation comprises a solubilizer, the solubilizer may consist of or comprise one or a combination of Cremophor EL, Tween, Brij C10, Kolliphor HS15, or Cremophor RH40. Preferably the solubilizer comprises Cremophor EL and/or Tween 80. The solubilizer may be present in the formulation in an amount of 5-60% by weight, such as at least about 7, 10, 15, 20 or 25%, and preferably may be present in an amount of 30-55%, 35-50%, or 40-45% by weight.

In some embodiments, the formulation further comprises an excipient. The excipient may consist of or comprise one or a combination of propylene glycol, ethanol, isopropanol, propylene carbonate, butylene glycol, n-butanol, and/or a polar oil, which may be isopropyl myristate. The excipient may be present in the formulation in an amount of 1-60% by weight, and preferably may be present in an amount of 20-55%, 25-50%, 30-45%, or 35-40% by weight.

In some embodiments, the formulation further comprises a stabiliser. The stabiliser may consist of or comprise a pH control agent. The pH control agent may comprise an organic acid, which may be citric acid, acetic acid, adipic acid, hydrochloric acid, N-hydroxysuccinic acid, lactic acid, malic acid, oxalic acid, propionic acid, ricinoleic acid, succinic acid, and tartaric acid. The stabiliser may be present in the formulation in an amount of up to about 3% by weight, and preferably may be present in an amount of 0.05-2.0%, 0.1-1.0%, 0.2-0.4%, such as about 0.3% by weight. Preferably, the formulation comprises citric acid in an amount of about 0.3%.

In some embodiments, a precipitation of the parasiticide is visible on the internal organs of the fish one week after intraperitoneal injection. In some embodiments the precipitate is also visible three weeks after intraperitoneal injection.

In some embodiments, a therapeutically effective concentration of the parasiticide is detectable in the tissues of the fish for at least 2 months (60 days) after intraperitoneal injection of the formulation. Preferably, a therapeutically effective concentration of the parasiticide is detectable in the tissues of the fish for at least 3 months (90, 100, or 110 days), 4 months (120, 130, or 140 days), 5 months (150, 160, or 170 days), 6 months (180, 200, 220 days), 8 months (220, 240, or 260 days), 10 months (300, 320, or 340 days), and preferably is detectable for at least 12 months (360 days) after intraperitoneal injection. Most preferably, a therapeutically effective concentration of the parasiticide is detectable in the tissues of the fish for at least 400 days, 500 days, 600 days, 700 days, or 750 days after intraperitoneal injection of the formulation.

In some embodiments, the formulation does not comprise a i-substituted azacycloalkan-2-one.

In some embodiments, the formulation may comprise: a parasiticide, which may be hexaflumuron; a non-aqueous polar solvent, which maybe DMSO in an amount of 5-25% or 10-20%, most preferably 15% by weight; a solubilizer, which may be Cremophor EL in an amount of, 20-80%, 30-70%, 40-60%, 45-55%, most preferably 50%, by weight; and an excipient, which may be propylene glycol in an amount of 20-50%, 25-45%, 30-40%, most preferably 35%, by weight. The formulation preferably also includes a stabiliser, which may be citric acid, in an amount of 0.1-0.5%, 0.2-0.4%, most preferably 0.3%. In these embodiments, the parasiticide may be present in an amount of 10-300 mg/ml, such as 50-260, 60-230, 70-190, 75-170, 80-150, 85-120, most preferably 90-100 mg/ml.

In some embodiments, the formulation may comprise: a parasiticide, which may be hexaflumuron; a non-aqueous polar solvent, which maybe DMSO in an amount of 5-25% or 10-20%, most preferably about 15%, by weight; a solubilizer, which may be Cremophor EL in an amount of 20-80%, 30-70%, 40-60%, 45-55%, most preferably about 50%, by weight; and a hydrophilic polymer, which may be a PEG or a PEG derivative, such as PEG300 or, preferably PEG400, in an amount of 20-50%, 25-45%, 30-40%, most preferably about 35%, by weight. The formulation preferably also includes a stabiliser, which may be citric acid, in an amount of 0.1-0.5%, or 0.2-0.4%, most preferably about 0.3%. In these embodiments, the parasiticide may be present in an amount of 10-300 mg/ml, such as 50-260, 60-230, 70-190, 75-170, 80-150, 85-120, most preferably about 90-100 mg/ml. For example, the formulation may comprise about 15% by weight DMSO, about 50% by weight Cr. EL, about 35% by weight PEG 400, about 0.3% citric acid, and about 90-100 mg/ml hexaflumuron.

In some embodiments, the formulation may comprise: a parasiticide, which may be hexaflumuron; a non-aqueous polar solvent, which maybe DMSO in an amount of 5-25%, or 10-20%, most preferably about 15% by weight; and a hydrophilic polymer, which may be a PEG or a PEG derivative, such as PEG300, or preferably PEG400, in an amount of 50-95%, or 70-90%, most preferably about 85% by weight. The formulation preferably also includes a stabiliser, which may be citric acid, in an amount of 0.1-0.5%, or 0.2-0.4%, most preferably about 0.3%. In these embodiments, the parasiticide may be present in an amount of 10-300 mg/ml, such as 50-260, 60-200, 70-180, 75-140, 80-130, 85-120 most preferably about 90-100 mg/ml. For example, the formulation may comprise about 15% by weight DMSO, about 85% by weight PEG 400, about 0.3% citric acid, and about 90-100 mg/ml hexaflumuron.

In some embodiments, the formulation may comprise: a parasiticide, which may be hexaflumuron; and a hydrophilic polymer, which may be a PEG or a PEG derivative, such as PEG300 or PEG400 in an amount of up to 50%, 60%, 70%, 80%, 90%, or 100% by weight. The formulation preferably also includes a stabiliser, which may be citric acid, in an amount of 0.1-0.5%, or 0.2-0.4%, most preferably about 0.3%. In these embodiments, the parasiticide may be present in an amount of 10-150 mg/ml, such as 15-120, 20-100, 25-90 or 30-80 mg/ml.

In some embodiments, the formulation may comprise:
(a) a hydrophilic polymer in an amount of about 80-90% by weight;
(b) a non-aqueous polar solvent in an amount of about 12-18% by weight;
(c) a stabilizer in an amount of about 0.2-0.4%; and,
(d) a chitin synthesis inhibitor in an amount of about 85-100mg/ml.

In some of these embodiments:
(a) the hydrophilic polymer may comprise PEG400 in an amount of about 85% by weight;
(b) the non-aqueous polar solvent may comprise DMSO in an amount of about 15% by weight;
(c) the stabilizer may comprise citric acid; and,
(d) the chitin synthesis inhibitor may comprise hexaflumuron in an amount of about 90mg/ml.

In accordance with a second aspect of the present invention there is provided a method of treating the parasitic infestation of fish. The method comprises administering a formulation in accordance with the first aspect to the fish by intraperitoneal injection.

In accordance with a third aspect of the present invention there is provided a formulation in accordance with the first aspect for use in the treatment of parasitic infestations of fish by intraperitoneal injection.

In accordance with a fourth aspect of the present invention there is provided the use of a formulation in accordance with the first aspect as an antiparasitic treatment of fish. The use comprises the intraperitoneal injection of the formulation.

### Detailed Description

An injectable formulation for fish, comprising a non-aqueous homogeneous solution of a parasiticide, has not previously been described. The claimed non-aqueous formulation may be administered to fish by means of intraperitoneal injection for the treatment of fish against parasites.

In the present context, the term "parasite" is intended to refer to any organism that lives on or in fish, or otherwise benefits at the expense of the host fish, for example, any organism that obtains some or all of its nutritional requirements from the fish. Parasites include both ectoparasites, that live or feed on the outer surface of the fish, and endoparasites, that live inside the body of the fish.

The term "parasiticide" refers to any substance that is capable of depleting the parasite population, for example by killing or preventing growth or reproduction of the parasites, or otherwise causing the loss or removal of parasites from the host fish. The substance may target a broad range of parasites, or may be specific for a small group of parasites, such as an individual type of parasite.

The terms "treating the parasitic infestation of fish", "treatment of parasitic infestations of fish", "treatment of fish", "treating fish against parasites", "controlling parasites", "treating parasites", and similar terms, are intended to refer to prophylactic or responsive treatment, such as the control, elimination, protection against, and/or prevention of infestations in fish with parasites. The treatment of parasite infestations encompasses reducing the mean number of parasites infecting each fish in a fish population. The control of parasite infestations encompasses preventing an increase in the mean number of parasites infecting each fish in a fish population.

### Fish

The formulation may be used to treat various different types of fish including food fish, breeding fish, aquarium, pond, river, reservoir fish of all ages occurring in freshwater, sea water, and brackish water. For example, bass, bream, carp, catfish, char, chub, cichlid, cobia, cod, eel, flounder, gourami, grayling, groupers, halibut, mullet, pangasius, plaice, pompano, roach, rudd, salmon, sole, tilapia, trout, tuna, whitefish, yellowtails, turbot, blue fin tuna, tench, amberjack, arowana, snakehead, puffers, croaker, rockfish, barramundi, meagre, sturgeon, lumpsucker, wrasse.

Of particular note, the formulation is suitable for treating fish of the order Samoniformes, Siluriformes, Perciformes, Cypriniformes, Tetraodontiformes, Osteoglossiformes, Acipenseriformes and Scorpaeniformes . For example, the claimed formulation may be used to treat salmon such as Atlantic and Pacific salmon, and trout such as rainbow trout and sea trout as well as sea bass, sea bream, tilapia, pangasius, turbot, and tuna.

### Parasites

The formulation may be used in the treatment of fish against parasites. This includes, in particular, parasites of the order Siphonostomatoida (lus), Dactylogyridea (Diplectanum), Mazocraeidea (such as Sparycotyle,and Heterobothrium), Hymenostomatida (freshwater white spot), Capsalidae (Benedenia), Dactylopodida (P. perurans), Cyclopoida, Parabodonida (Cryptobia spp.), Scuticocilitida (ciliates), Gluegeida (L. salmonae), Bivalvulida (such as Myxobolus, Ceratomyxa and H. ictaluri), Monopisthocotylea (gyrodactylus), Strigeatida (blood flukes), Botriocephalidea (tapeworms), Spirurida (nematodes), Arguloida (carp lice infreshwater) and Ascaridida (Anisakis) and genus Cryptocaryon (white spot in seawater), Diplostomum (eye fluke in freshwater), and Enteromyxum (E. leei).

This includes the following families: Caligidae, Cecropidae, Dichelesthiidae, Lernaeopodidae, Pandaridae, Pennellidae, Sphyriidae, Lernaeidae, Bomolochidae, Chondracanthidae, Ergasilidae, Philichthyidae, and Argulidae. Of particular interest are parasites of the genera *Dissonus, Caligus* (including in particular, *C. curtus, C. elongatiis, C. clemensi, C. rogercresseyii*)*,* and *Lepeophtheirus* (including *L. salmonis*). Possible localisations of parasites within the fish are skin, gills, intestines, and other internal organs as well as other locations such as the eyes and/or the mouth.

### Parasiticides

The chitin synthesis inhibitors for use in the formulation is hexaflumuron.

### Parasiticide concentration

The parasiticide may be used at any suitable concentration or amount. For example, when the inhibitor is a chitin synthesis inhibitor such as hexaflumuron, the inhibitor may be used in the amount of 5-300mg/ml.

In one example, the formulation may comprise: a parasiticide; a non-aqueous polar solvent, which may be DMSO; a solubilizer, which may be Cremophor EL; and an excipient, which may be propylene glycol. In these formulations, the parasiticide may be present in an amount of 10-300 mg/ml, such as 20-250, 30-200, 40-150, 50-120, 70-115, 80-110, 85-105, most preferably 90-100 mg/ml, such as about 90 mg/ml or about 100 mg/ml.

In another example, the formulation may comprise: a parasiticide, which may be hexaflumuron; and a hydrophilic polymer, which may be a PEG or a PEG derivative, such as PEG300 or PEG400 in an amount of up to 50%, 60%, 70%, 80%, 90%, or 100% by weight. The formulation preferably also includes a stabiliser, which may be citric acid, in an amount of 0.1-0.5%, or 0.2-0.4%, most preferably about 0.3%. In these formulations, the parasiticide may be present in an amount of 10-150 mg/ml, such as 15-120, 20-100, 25-90 or 30-80 mg/ml.

In another example, the formulation may comprise: a parasiticide; a non-aqueous polar solvent, which may be DMSO; a solubilizer, which may be Cremophor EL; and a hydrophilic polymer, which may be a PEG or a PEG derivative, such as PEG300 or PEG400. In these formulations, the parasiticide may be present in an amount of 10-300 mg/ml, such as 20-250, 30-200, 40-150, 50-120, 70-115, 80-110, 85-105, most preferably 90-100 mg/ml, such as about 90 mg/ml or about 100 mg/ml.

In yet another example, the formulation may comprise: a parasiticide; a non-aqueous polar solvent, which may be DMSO; and a hydrophilic polymer, which may be a PEG or a PEG derivative, such as PEG300 or PEG400. In these formulations, the parasiticide may be present in an amount of 10-300 mg/ml, such as 20-250, 30-200, 40-150, 50-120, 70-115, 80-110, 85-105, most preferably 90-100 mg/ml, such as about 90 mg/ml or about 100 mg/ml.

The formulation may comprise a combination of two or more parasiticides, such as two or more parasiticides with different modes of action. When the formulation comprises two or more parasiticides, the inhibitors may be used such that the total combined inhibitor concentration is in the range 5-300mg/ml. For example, the total combined inhibitor concentration may preferably be in the range of 10-250mg/ml, and most preferably in the range of 20-225mg/ml or 25-200mg/ml, and most preferably in the range of 50-175mg/ml or 75-150mg/ml, such as about 80, 90, 100, 110, or 120mg/ml. Most preferably, the total combined inhibitor concentration may be about 90-100mg/ml.

Within the treated fish, the parasiticide is preferably distributed in a pharmacologically effective concentration to all tissues and organs, including mucus, skin, gills, and intestines.

### Intraperitoneal injection

Parasiticides such as chitin synthesis inhibitors have previously been administered to fish orally. For example, chitin synthesis inhibitors such as hexaflumuron have been added to pre-manufactured fish feed or pellets or mixed with the other components in fish feed before making pellets. Chitin synthesis inhibitors have also previously been administered by means of addition to the water in which the fish are contained. This method of administration is very simple and labour efficient, and has the added advantage that the inhibitor may be able to act directly on any parasites which are externally accessible.

The present formulation, however, is suitable for administration to fish by means of intraperitoneal injection. This route of administration is more technically challenging, labour intensive, and expensive than other methods of administration and therefore would not be the most straightforward and obvious method of treating fish with insecticide.

### Formulation

Intraperitoneal injection involves injecting a formulation onto the surface of the internal organs of the fish. As a result, the effects, and in particular, the toxicity of injected formulations are difficult to predict, and may be different when the composition is delivered to the fish by intraperitoneal injection in comparison to administration by other means. The effects of solvents in particular, when injected into the peritoneal cavity of a fish, have not previously been fully investigated in large scale studies. For example, solvents may damage the surface of the internal organs or cause other internal injuries to the fish. For this reason, previous formulations for intraperitoneal injection have generally only been produced in the form of aqueous preparations. For example, WO 99/063824 discloses an aqueous preparation of a chitin synthesis inhibitor.

However, the water-solubility of some parasiticides, including chitin synthesis inhibitors, is very low. For example, the water solubility of hexaflumuron has been found to be about 2.7 x 10⁻⁵ mg/ml. As a result, if hexaflumuron is prepared in the form of an aqueous solution it delivers poor performance, since the concentration of hexaflumuron is very low due to the low solubility of hexaflumuron in water.

In order to address this problem, WO 2014/016252 discloses the production of an aqueous formulation comprising nanoparticles of the parasiticides hexaflumuron or lufenuron in the form of a suspension.

It has now been found, however, that there are drawbacks with formulations comprising a suspension or emulsion. For example, in a water-in-oil emulsion in which particulate hexaflumuron is dispersed in the continuous oil phase, the hexaflumuron particles may sediment during production, storage and use, leading to an inhomogeneous product. Moreover, the opacity of the emulsion hides the extent of the sedimentation.

The claimed formulations are homogenous and do not comprise a suspension or a plurality of liquid phases, such as an emulsion. As a result, they have been found to be physically stable over a prolonged period of time, and under various different storage conditions and temperatures. This offers a significant and important advantage over suspension and multiphasic formulations, which are known to be intrinsically unstable and, therefore, challenging to store for prolonged time.

The formulation comprises a parasiticide in homogeneous non-aqueous solution, wherein the parasiticide is entirely dissolved in the solvent, giving a homogeneous, thermodynamically stable liquid formulation. The parasiticide is not present in particulate form, for example, comprising a suspension. The formulation is preferably a non-micellar or substantially non-micellar solution and the parasiticide is not contained within a dispersion. The formulation comprises a single non-aqueous phase, and does not comprise an emulsion.

It has been found that some parasiticides, including chitin synthesis inhibitors, such as hexaflumuron, are essentially insoluble in both water and non-polar solvents such as heptane. In addition, in many solvents, and in particular organic solvents, hexaflumuron has been found to be chemically unstable.

Surprisingly, however, parasiticides have now been found to have a degree of solubility in certain polar solvents including Dimethyl sulfoxide (DMSO), Dimethylformamide (DMF), Dimethylacetamide (DMA), tetraglycol, n-methyl-2-pyrrolidone (NMP), acetone and ethyl acetate. In particular, it has been found to be possible to formulate parasiticides, including chitin synthesis inhibitors such as hexaflumuron, in such a way that they have sufficient solubility and stability in these polar solvents to be suitable for intraperitoneal injection.

### Polar solvents

A solvent is considered to be a polar solvent if the solvent molecules have a permanent separation of positive and negative charges, or the centres of positive and negative charges do not coincide. Polar solvents have high dielectric constants, for example, a solvent with a dielectric constant above about 15.0 is generally considered to be a polar solvent.

In some embodiments, the formulation comprises a parasiticide in solution in a polar solvent. For the avoidance of doubt, in relation to the claimed formulation, the term "polar solvent" does not encompass the use of water as a solvent, and for clarity, the polar solvent may therefore be referred to as a "non-aqueous polar solvent". In practice, no water is added during the production of the formulations, but as the skilled person would appreciate, it is nevertheless possible that a small amount of water may unavoidably be present, for example as a result of being adsorbed from the atmosphere. Thus, the present formulation is a non-aqueous formulation and contains at most 4%, 3%, or 2%, preferably at most 1.5%, 1% or 0.5% water. In the present context, the term "non-aqueous" refers to formulations which include at most only a very small amount of water, which is not added to the formulation, but may be derived, for example, from the atmosphere. For example, some non-aqueous polar solvents, such as DMSO, are known to by hygroscopic under certain conditions and may therefore cause small amounts of water to accumulate in the formulation, or in the solvent prior to the production of the formulation. For the purposes of clarity, the formulations comprise no water other than that which may be present in the constituent agents due to adsorption.

Examples of polar solvents that may be used individually or in combination in some embodiments of the claimed formulation include acetone, tetraglycol, ethyl acetate, DMF, DMA, DMSO, NMP, ethanol, isopropanol, glycerin, ethylene glycol, diethylene glycol, 1-propanol, 1-butanol, 2-butanol, acetonitrile and methanol.

The polar solvent may be a polar aprotic solvent such as DMF, DMA, or DMSO. For example, parasiticides, including chitin synthesis inhibitors such as hexaflumuron, have been found to be sufficiently soluble in polar aprotic solvents to enable the production of a suitable formulation for use in intraperitoneal injection. The solubility of hexaflumuron in DMSO, for example, has surprisingly been found to be greater than 300mg/ml. As a result, the preferred non-aqueous polar solvent is DMSO.

In some embodiments, the non-aqueous polar solvent does not comprise NMP, tetraglycol, and/or acetone. Propylene glycol is not considered to be a non-aqueous polar solvent, and because it is a poor solvent for parasiticides such as hexaflumuron, propylene glycol cannot be used as a solvent in the present formulation. As described below, however, propylene glycol may be used as an excipient in combination with a non-aqueous polar solvent such as DMSO and/or a hydrophilic polymer such as PEG.

### Toxicity

The claimed formulations are suitable for use by intraperitoneal injection, and therefore do not induce mortality when administered to fish in this way.

It is difficult to predict the effects, and in particular, the toxicity of formulations administered to fish by intraperitoneal injection, and these effects may be different to those observed when the same formulation is administered by other means, or to different animals.

For example, although DMSO has previously been shown to have low toxicity when orally administered to various different types of fish, including salmonids (Benville et al, Toxicology And Applied Pharmacology 12, 156-178 (1968)), it was not previously known whether polar solvents would be toxic to fish if administered by means of intraperitoneal injection together with a parasiticide such as a chitin synthesis inhibitor. It has now surprisingly been found that such formulations are toxic to the fish, and that this toxicity is due to the polar solvent and not to the parasiticide. The discovery of this previously unrecognised problem is significant and unfortunate because it has also now been found that formulations comprising non-aqueous polar solvents demonstrate significantly superior uptake and bioavailability of parasiticide than equivalent emulsion or aqueous suspension formulations.

Toxicity has been found to be related to the size and weight of the fish, so depending on the fish population to be treated, the level of polar solvent that may be used without inducing excessive mortality may differ. For example, larger fish may be able to tolerate greater amounts and concentrations of polar solvent than smaller fish. The claimed formulations are non-toxic and produce no mortality.

Toxicity may be measured by any applicable method, and suitable methods are known to the skilled person. For example, the formulation, which is not toxic to fish, may induce at most 2% mortality in the fish population that has been treated. More preferably, the level of mortality is less than 1%, such as less than 0.5% compared to placebo treated control group.

As the skilled person will be aware, the toxicity of a formulation can be determined in a number of ways. Some methods may involve trials involving large numbers of fish. Therefore, to avoid the need to test large numbers of fish, a model test for toxicity of the formulations involving much smaller numbers may be used. The use of a model test such as that described below provides the further advantage that it removes complicating factors such as the size and sensitivity of the fish tested, because small fish are often more susceptible to a given treatment dosage and intraperitoneal injection than larger fish. In this way, the model test allows a standardised comparison of the toxicity of different formulations to be obtained.

In the model test, a small number of Atlantic salmon, of 24-26 grams in size, such as 30 fish per group, are treated at a water temperature of about 17 degrees C. One group of fish is treated (by intraperitoneal injection) with a large dosage volume (0.2ml) of the subject formulation, and a second group is treated in the same way with a regular dosage volume (0.05ml) of the subject formulation. Additionally, two control groups are treated in an identical manner to the subject groups, but with a control formulation, such as PBS, instead of the subject formulation. The fish are monitored and the accumulated mortality over seven days is recorded. The subject formulation is then considered to be non-toxic if two conditions are met. The first condition is that there is no increased mortality in the subject group versus the relevant control group when the formulation is administered in an amount of 0.05ml. The second condition is that there is at most a 40% increase in mortality in the subject group versus the relevant control group when the formulation is administered in an amount of 0.2ml.

Generally, when the polar solvent is a polar aprotic solvent such as DMSO, the formulation comprises 5-40% (w/w) polar solvent. Preferably, the formulation comprises less than 35%, less than 30%, less than 25%, or less than 20% (w/w) polar solvent, such as, for example, 7-35%, 10-30%, 12-25% or 15-20% (w/w) polar solvent, such as about 5%, 10%, 15%, 20%, or 25% (w/w) polar solvent.

### Solubilizer

Solubilization is a process in which the solubility of a compound is increased by the formation of micellar structures after addition of a solubilizer.

The term solubilisation is almost exclusively used for aqueous systems. In aqueous pharmaceutical formulations, for instance, solubilizers are often used to facilitate dissolution of poorly soluble compounds. In non-aqueous formulations, on the other hand, solubilizers are mainly added in order to have a function during the use of the products in aqueous media and not in the formulation *per se.* For instance, solubilizers are essential components in self-emulsifying drug delivery systems, SEDDS (Singh B. et al. Crit Rev Ther Drug Carrier Syst. 2014;31(2):121-185). SEDDS are isotropic mixtures of non-aqueous solvents, solubilizers and co-solvents to form fine oil-in-water emulsions when introduced in aqueous media.

While the process of solubilization is well described in aqueous systems, solubilization in homogenous, entirely non-aqueous systems *per* se is less well understood. Basically, there is no clear rational reason for adding a solubilizer to a non-aqueous formulation, unless the solubilizer has a function during use in aqueous media as described above. Hence, in non-aqueous formulations such as those described in the present invention, the use of a solubilizer would not be considered. The claimed formulations are preferably non-micellar, or substantially non-micellar solutions and the parasiticide is preferably not substantially associated with, or contained within, micelles. For clarity, the formulations of the present application that do not comprise a solubilizer are non-micellar.

It has now been surprisingly and advantageously discovered that it is possible to produce a formulation comprising a non-aqueous polar solvent, and that the level of non-aqueous polar solvent required in the formulation may be reduced below toxic levels if a solubilizer is also included. Thus, it has now been discovered that the surprising solution to the previously unrecognised problem of the toxicity of intraperitoneally injected non-aqueous polar solvents is to formulate the non-aqueous polar solvent together with a solubilizer.

In the present context, the term "solubilizer" refers to any substance that is capable of increasing the solubility of the parasiticide in the polar solvent or in a mixture of polar solvent and excipient, or polar solvent and hydrophilic polymer. The solubilizer may be an amphiphilic molecule or a surfactant, and could be cationic, anionic or non-ionic, non-polymeric or polymeric.

Various solubilizers may be used in the formulation in combination with a non-aqueous polar solvent and/or a hydrophilic polymer, and for example, the solubilizer may be selected from the list of solubilizers or emulsifiers given in the Handbook of pharmaceutical additives, M. Ash and I. Ash, Gower publishing limited, 1996, page 900-903 or 874-880, respectively.

The solubilizer may comprise a mixture of two or more different solubilizers.

Suitable solubilizers include, for example: polyoxyethylene sorbitane fatty acid esters such as polysorbate 20 (Tween 20), polysorbate 21, polysorbate 40, polysorbate 60, polysorbate 61, polysorbate 65, polysorbate 80 (Tween 80), polysorbate 81, polysorbate 85 and polysorbate 120; polyoxyethylene castor oil derivatives such as Polyoxyl 5 castor oil, Polyoxyl 9 castor oil, Polyoxyl 15 castor oil, Polyoxyl 35 castor oil (Cremophore EL), Polyoxyl 40 castor oil, Polyoxyl 40 hydrogenated castor oil, polyoxyl 60 castor oil, Polyoxyl 60 hydrogenated castor oil, Polyoxyl 100 castor oil, Polyoxyl 100 hydrogenated castor oil, Polyoxyl 200 castor oil and Polyoxyl 200 hydrogenated castor oil; poloxamers (polyoxyethylene - polyoxypropylene copolymers) such as poloxamer 124, poloxamer 188, poloxamer 237, poloxamer 338 and poloxamer 407; polyoxyethylene alkyl ethers such as Cetomacrogol 1000, Polyoxyl 6 cetostearyl ether, Polyoxyl 20 cetostearyl ether, Polyoxyl 25 cetostearyl ether, Polyoxyl 2 cetyl ether, Polyoxyl 10 cetyl ether, Polyoxyl 20 cetyl ether, Polyoxyl 4 lauryl ether, Polyoxyl 9 lauryl ether, Polyoxyl 23 lauryl ether, Polyoxyl 2 oleyl ether, Polyoxyl 10 oleyl ether, Polyoxyl 20 oleyl ether, Polyoxyl 2 stearyl ether, Polyoxyl 10 stearyl ether, Polyoxyl 21 stearyl ether and Polyoxyl 100 stearyl ether; polyoxylglycerides such as Caprylocaproyl polyoxylglycerides, Lauroyl polyoxylglycerides, Linoleoyl polyoxylglycerides, Oleoyl polyoxylglycerides and Stearoyl polyoxylglycerides; and sorbitan fatty acid esters like Sorbitan diisostearate, Sorbitan dioleate, Sorbitan monoisostearate, Sorbitan monolaurate, Sorbitan monooleate (Span 80), Sorbitan monopalmitate, Sorbitan monostearate, Sorbitan sesquiisostearate, Sorbitan sesquioleate, Sorbitan sesquistearate, Sorbitan triisostearate, Sorbitan trioleate and Sorbitan tristearate; macrogol 15 hydroxystearate; cetostearyl alcohol and cetyl alcohol.

Solubilizers that have been found to be particularly suitable for use in some embodiments include, for example, Tween, Brij C10, Kolliphor HS15, or Cremophor RH40. Especially preferred solubilizers include Tween 80 and Cremophor EL.

When the formulation comprises a solubilizer, the solubilizer is generally used in the amount of 5-60% (w/w). Preferably, the formulation comprises 10-55% (w/w) solubilizer, more preferably 15-50% (w/w) solubilizer, such as, for example, about 20, 25, 30, 35, 40, 45, 50 or 55% (w/w) solubilizer.

When the formulation comprises Cremophor EL as the solubilizer, it is preferably present in the formulation in an amount of at least about 5, 7, 10, 12, 15, 18, 20 or 25%, such as 30-60%, such as about 35, 40, 45, 50 or 55%.

### Hydrophilic Polymer

As an alternative, or in addition, to the use of a solubilizer in the formulation, it has also surprisingly and advantageously been found that the toxic effects of intraperitoneally injected non-aqueous polar solvents can be overcome if the polar solvent is formulated together with a hydrophilic polymer. Thus, it has surprisingly and advantageously been discovered that it is possible to produce a parasiticide formulation that is suitable for intraperitoneal injection of fish, comprising a non-aqueous polar solvent, and that the level of polar solvent required in the formulation may be reduced below toxic levels if a hydrophilic polymer is also included.

Furthermore, it has surprisingly and advantageously been found that the solubility of parasiticides, including chitin synthesis inhibitors such as hexaflumuron, in hydrophilic polymers is sufficiently high that formulations suitable for intraperitoneal injection can surprisingly be produced without the need for any additional agents, such as a polar solvent and/or a solubilizer. This provides at least the important advantage that the possibility of any toxic effects of non-aqueous polar solvents can optionally be entirely avoided.

Moreover, it has surprisingly been found that using a hydrophilic polymer, a formulation can be produced that is not toxic to fish and demonstrates a significant and highly advantageous depot effect. This depot effect has been found to arise as a result of the precipitation of parasiticide in the peritoneal cavity. In a highly unexpected manner that provides an extremely advantageous new tool for the user, it has been found that formulations comprising a solubilizer do not result in the precipitation of parasiticide in the peritoneal cavity, whereas a precipitate is observed following the intraperitoneal injection of formulations that comprise a hydrophilic polymer, and do not comprise a solubilizer. These formulations, comprising a hydrophilic polymer together with little or no solubilizer, which have been found to provide a depot effect, show an advantageously increased half-life of parasiticide in the tissues of the treated fish. In contrast, upon intraperitoneal injection to fish, formulations comprising a solubilizer have a shorter half-life of parasiticide, but a higher peak tissue concentration. Thus, the user can easily control the parasiticide half-life and peak concentration by adjusting the composition of the formulation, and specifically by controlling the solubilizer and/or hydrophilic polymer content of the injectable formulations.

This long-lasting duration of efficacy is a major advantage as it reduces the frequency with which parasiticides must be administered to fish. Indeed, in some cases, the half-lives may be sufficient for one 0.05ml administration to protect a fish for the entire marine growth-period.

Hydrophilic polymers contain polar or charged functional groups, rendering them soluble in water and/or in polar solvents. Various hydrophilic polymers may be used in the formulation, optionally in combination with a non-aqueous polar solvent and/or solubilizer, or in the absence of any other solvent or solubilizer.

The hydrophilic polymer may be a polymer that is capable of hydrogen bonding.

Preferably the hydrophilic polymer has an average molecular weight of 50-2000, such as between 100-1800, or 150-1600.

The hydrophilic polymer may be a polyalkoxylated alcohol, or a derivative of a polyalkoxylated alcohol. Preferably, the hydrophilic polymer is a polyalkoxylated diol or a derivative of a polyalkoxylated diol. For example, the hydrophilic polymer may be a polyalkylene glycol or a derivative of a polyalkylene glycol.

Suitable hydrophilic polymers include, in particular, PEGs and PEG derivatives and PPGs and PPG derivatives.

Examples of PEG derivatives that may be used include: monofunctional PEGs, such as poly(ethylene glycol) methylether; homobifunctional PEGs, such as poly(ethylene glycol) dimethylether, poly(ethylene glycol) diglycidyl ether, or poly(ethylene glycol) bis(carboxymethyl) ether; heterobifunctional PEGs, such as poly(ethylene glycol) tetrahydrofurfuryl ether; multi-arm PEGs or star-PEGs, such as trimethylolpropane ethoxylate, or glycerol ethoxylate.

Examples of PPG derivatives that may be used include: monofunctional PPGs, such as poly(propylene glycol) monobutyl ether; and homobifunctional PPGs, such as poly(propylene glycol) diglycidyl ether.

The hydrophilic polymer may be a polyether such as glycerol propoxylate.

Preferably the hydrophilic polymer is PEG or a PEG derivative. For example, the hydrophilic polymer may be PEG having a molecular weight of between 200 and 1000. Preferably the hydrophilic polymer is PEG 300, PEG 400, or PEG 600.

The hydrophilic polymer may comprise a combination of two or more hydrophilic polymers.

Preferably the hydrophilic polymer is not toxic to fish when administered by intraperitoneal injection.

The hydrophilic polymer may be present in the formulation as the only solvent. In some embodiments, the hydrophilic polymer may be present in the formulation in an amount of 5-100% by weight.

The hydrophilic polymer may be present in the formulation in combination with another solvent, such as a non-aqueous polar solvent. In this case, the hydrophilic polymer may be present in the formulation an amount of 10-99%, 15-98%, 20-97%, 25-96%, 30-95%, 35-94%, 40-93%, 45-92%, 50-91%, 55-90%, 60-89%, 65-88%, 70-87%, 75-86%, preferably about 80-85%by weight.

### Excipient

The formulation may comprise an excipient. In the present context, the term "excipient" is used to refer to any inert, non-toxic substance which is added to the formulation, for example, as a diluent to allow the formulation to be prepared to the desired consistency, form or concentration.

Any suitable excipient may be used in the formulation, and the formulation may comprise one or a combination of excipients. For example, the excipient or mixture of excipients could be selected from, but not restricted to, the list of carriers/vehicles or solvents given in Handbook of pharmaceutical additives, M. Ash and I. Ash, Gower publishing limited, 1996, page 862-863 or 903-904, respectively.

Suitable excipients include, for example, polar, low-molecular weight substances like propylene glycol, butylene glycol, propylene carbonate, ethanol, isopropyl alcohol, glycerol, butanol and ethyl lactate.

The excipient for use in the formulation may include one or more polar oils. Polar oils contain heteroatoms that give the molecules a dipole moment, and this provides the substances with unique solubility properties when compared with nonpolar oils such as mineral oil.

Polar oils which may be suitable for use in the formulation include, for example: fatty alcohols such as myristyl alcohol, oleyl alcohol and stearyl alcohol; esters such as isopropyl myristate, isopropyl palmitate and ethyl oleate; triglycerides such as tricaprylin, medium-chain tryglycerides and triolein; and coconut oil.

Generally, the formulation may comprise about 5-70% (w/w) of excipient. Preferably one or a combination of excipients is included in the formulation in a total amount of 10-60% (w/w), 20-55% (w/w), or 30-50% (w/w), such as about 35, 37.5, 40, 42.5, or 45% (w/w).

### Stabilisers

It has surprisingly now been discovered that the chemical stability of hexaflumuron in the present non-aqueous formulations can be dramatically increased by including a stabiliser. The stabiliser may be a pH control agent, such as for example, citric acid. Thus, in some circumstances, it may be advantageous to include a stabiliser, such as a pH control agent, in the formulations.

As the skilled person would be aware, the term "pH" only makes sense for aqueous media and not for non-aqueous solutions such as the present formulations. Therefore, in the present context the terms "pH control agent", "pH modifier", and similar terms, are used to refer to any substance, which, when used in an aqueous solution, is potentially capable of controlling the pH, such as buffering and/or adjusting the pH. Such agents have surprisingly been found to increase the chemical stability of hexaflumuron in the present non-aqueous formulations.

A pH control agent may be included in the formulation in an amount of up to about 3 volume%, such as 0.05-2.0 volume%, or 0.1-1.0 volume%. Preferably, the pH control agent may be included in the formulation in an amount of about 0.1, 0.2, 0.3, 0.4, or 0.5 volume%, and most preferably may be included in an amount of 0.3 volume%.

Various stabilisers, which may be pH control agents, may be used in the formulation. For example, the pH control agent for use in the formulation may be selected from, but not restricted to, the list of pH control agents given in Handbook of pharmaceutical additives, M. Ash and I. Ash, Gower publishing limited, 1996, page 896. Specifically, suitable pH control agents for use in the formulation have been found to include, for example, organic acids like citric acid, acetic acid, adipic acid, hydrochloric acid, N-hydroxysuccinic acid, lactic acid, malic acid, oxalic acid, propionic acid, ricinoleic acid, succinic acid and tartaric acid.

### Other agents

Additional agents may be included in the formulation, as required.

The formulation may include a freezing point lowering agent. Suitable freezing point lowering agents include, for example, propylene glycol and ethanol.

The formulations of the present invention may further comprise a preservative. Any suitable preservative or combination of preservatives may be used, including, for example, chlorobutanol, benzyl alcohol, a paraben (such as methylparaben or propylparaben), sorbic acid, phenoxyethanol, or thiomersal.

The preservative may be included in the formulation in an amount of from 0.001 % to 2.5 % (w/v), such as from 0.002 to 1.5% (w/v).

The formulations of the present invention may further comprise a crystal growth inhibitor. Any suitable crystal growth inhibitor or combination of crystal growth inhibitors may be used, including, for example, a mono- or disaccharide, a sugar alcohol, a glycol, glycerol, an at least partly water-soluble salt, and mixtures thereof. Examples are dextrose, sucrose, fructose, mannose, lactose, trehalose; mannitol, sorbitol, xylitol; ethylene glycol, propylene glycol; glycerol; sodium chloride, potassium chloride, magnesium chloride. Preferred crystal growth inhibitors include mannitol, sorbitol, dextrose, lactose, sucrose, trehalose, propylene glycol, glycerol and sodium chloride. The amount of crystal growth inhibitor in the formulation of the present invention may be, for example, 0.1-20 % (w/v), preferably from 0.25-15% (w/v), and in particular from 0.5-10% (w/v).

The formulations of the present invention preferably do not comprise a 1-substituted azacycloalkan-2-one.

### Use of the formulations

It has surprisingly been found that the claimed antiparasitic formulations are significantly more effective at treating fish against parasites than previous preparations of parasiticides, including those administered by injection, in feed, or by means of bath treatment. In particular, when the formulations comprise a chitin synthesis inhibitor, they have been found to demonstrate increased bioavailability relative to previous preparations of chitin synthesis inhibitors. As a result, the dose per fish as well as the volume to be injected may be reduced. This improves the safety profile of the product. Also, the therapeutic effect may be prolonged due to increased tissue concentrations. Products with an improved safety profile and prolonged effect present obvious economic benefits to the fish farmers. It may also be environmentally beneficial since less of the active pharmaceutical ingredient is released to the environment.

Moreover, since the active compound is in solution, it also has much better long term physiochemical stability than a suspension such as that described in WO 2014/016252.

The claimed formulations are suitable for intraperitoneal injection to fish in any suitable manner. For example, in the case of salmonids, one treatment schedule comprises treating the fish during the initial fresh water phase before transfer to sea. In addition, or alternatively, the treatment may be performed whilst the fish are already at sea.

Generally, the total volume of the antiparasitic formulation that is injected into the fish is about 0.01-2ml, such as, for example, 0.05ml, or 0.1ml. The overall amount of parasiticide injected into the fish is preferably from 0.00010 to 250 mg per kg of fish biomass, more preferably from 0.0015 to 150 mg per kg of fish biomass, even more preferably from 0.2 to 120 mg/kg fish biomass. For example, when the parasiticide is a chitin synthesis inhibitor such as hexaflumuron, the amount of inhibitor that may be administered may be from 1 to 75 mg/kg of fish biomass, such as 5-50, or 10-30 mg/kg of fish biomass.

The claimed antiparasitic formulations also have a number of other advantages. In particular, it has surprisingly been found that in the present formulations the chitin synthesis inhibitors may be highly chemically stable, providing a long shelf life.

The fish absorbs the parasiticide, and the concentration of the parasiticide is preferably maintained and detectable at a therapeutically effective concentration within the blood, fillet and skin of the fish for an extended period of time. Thus, in addition to a direct treatment effect, the formulation generally also protects fish from new attacks by parasites for an extended period of time after administration. The depletion rate of the parasiticide, and hence the period of protection, largely depends on the nature of the formulation. As described above, the present formulations have been unexpectedly and advantageously found to provide a controllable depot effect, providing the skilled person with a valuable tool for controlling both the peak concentration of parasiticide in the fish tissue and the duration of protection provided by the parasiticide.

When administering the claimed formulation, a therapeutically effective concentration of the parasiticide may be detected in the tissues of the fish for at least 2 months (60 days) after administration, thus indicating an effective protection of the fish against parasites for a prolonged period of time. Preferably, the claimed formulation provides protection for the fish from new infestations of parasites for a period of at least 3 months (90 days), and most preferably, the fish are protected for at least 5 months (150 days), at least 6 months (180 days), at least 8 months (240 days), at least 10 months (300 days), at least 12 months (360 days), at least 15 months (450 days), at least 18 months (540 days) , at least 20 months (600 days), at least 24 months (720 days), or at least 25 months (750 days) following administration. It has been shown that the protection period of the fish against parasites corresponds very well with the observed levels of the chitin synthesis inhibitor in the fish fillet, skin or blood.

It has now surprisingly been found that following injection with the claimed antiparasitic formulations, particularly those comprising a hydrophilic polymer, and that comprise a low concentration of solubilizer, or no solubilizer, precipitated parasiticide may be observed in the visceral cavity. The presence of precipitated parasiticide has been found to correlate with an extremely advantageous depot effect in which the half-life of parasiticide in the fish tissues is significantly increased, relative to formulations in which no precipitate is observed. In this case, a therapeutically effective concentration of the parasiticide may be detectable in the tissues of the fish for at least 6 months (180 days), at least 8 months (240 days), at least 10 months (300 days), at least 12 months (360 days), at least 15 months (450 days), at least 18 months (540 days) , at least 20 months (600 days), at least 24 months (720 days), or at least 25 months (750 days) following intraperitoneal injection of the formulation.

The extended post-treatment protection period provided by the claimed formulation is especially advantageous since the need for repeated treatments is substantially reduced. This reduces pollution by therapeutic substances in the environment and is also cost effective and labour saving for the fish farmer.

Moreover, the depot effect can advantageously be easily controlled by adjusting the composition of the injectable formulations.

In some circumstances, it may be desirable to use the claimed antiparasitic formulation with an additional antiparasitic agent, such as an additional insecticide. Suitable further insecticides that may be used in combination with the claimed antiparasitic formulation are, for example, hydrogen peroxide; formaldehyde; an organophosphate such as trichlorfon, malathion, dichlorvos or azamethiphos; a macrocyclic lactone such as ivermectin, or emamectin benzoate; a pyrethroid such as cypermethrin; or a spinosyn such as spinosad.

### Pharmaceutical kits

The invention also provides a pharmaceutical pack or kit comprising one or more containers filled with the formulation of the invention. Additionally provided in the kit is notice in the form prescribed by a governmental agency regulating the manufacture, use or sale of the pharmaceuticals, and an instruction for use of the pharmaceutical.

### Examples

The invention will now be explained in further detail in the following Examples, which demonstrate the development of the claimed antiparasitic formulations for intraperitoneal injection of fish.

### Example 1

Hexaflumuron was studied as an example parasiticide that is insoluble in water.

The solubility of hexaflumuron in common solvents methanol, water, and xylene has previously been investigated and the results are shown in table 1. For the purposes of producing a stable solution suitable for intraperitoneal injection, hexaflumuron is not sufficiently soluble in these solvents.

**Table 1**

| **Solvent** | **Hexaflumuron Solubility (mg/ml)** |
|---|---|
| Water | 2.7 x 10⁻⁵ |
| Methanol | 11.3 |
| Xylene | 5.20 |

Tomlin, C.D.S. (ed.). The Pesticide Manual - World Compendium, 11 th ed., British Crop Protection Council, Surrey, England 1997, p. 676

In order to produce a formulation in which hexaflumuron was present in stable solution, the solubility of hexaflumuron in various other solvents was investigated. Solubility was determined by adding each solvent gradually to a specific mass of hexaflumuron until complete dissolution was observed. The resulting solubilities are given in table 2.

**Table 2**

| **Solvent** | **Hexaflumuron Solubility (mg/ml)** |
|---|---|
| Acetone | >130 |
| Tetraglycol | >75.0 |
| Ethyl acetate | >75.0 |
| Isopropyl myristate | <1.0 |
| n-heptane | <0.5 |
| Propylene glycol (PG) | <0.5 |
| Paraffin Light Liquid | <0.10 |
| DMSO | >300 |
| NMP | >300 |
| DMF | >300 |
| DMA | >300 |

The solubility of hexaflumuron was found to be very low in non-polar solvents such as heptane.

However, the solubility of hexaflumuron was found to be surprisingly high in some polar solvents, such as DMSO, NMP, DMF and DMA.

### Example 2

Formulations of a parasiticide prepared in a polar solvent were tested for safety for intraperitoneal injection into Atlantic salmon.

Atlantic salmon parr (average size: 35.5 g) kept in freshwater (17°C) were injected with one of 10 formulations containing DMSO as an example polar solvent, an excipient comprising propylene glycol (PG), and an amount of hexaflumuron varying from 0 to 100 mg/ml (see table 3). Each of the 10 groups contained 30 fish.

**Table 3**

| **Group** | **Injection Volume (ml)** | **Hexaflumuron (mg/ml)** | **DMSO (mass %)** | **PG (mass%)** |
|---|---|---|---|---|
| 1 | 0.05 | 10 | 85 | 15 |
| 2 | 0.05 | 15 | 85 | 15 |
| 3 | 0.05 | 20 | 85 | 15 |
| 4 | 0.05 | 25 | 85 | 15 |
| 5 | 0.05 | 50 | 85 | 15 |
| 6 | 0.05 | 100 | 85 | 15 |
| 7 | 0.05 | 0 | 85 | 15 |
| 8 | 0.10 | 25 | 85 | 15 |
| 9 | 0.20 | 25 | 85 | 15 |
| 10 | 0.20 | 0 | 85 | 15 |

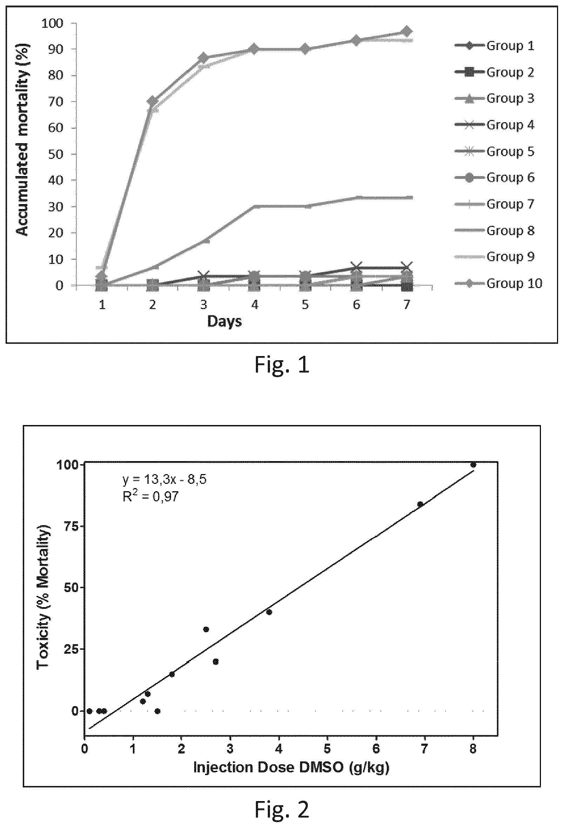

Figure 1. Mortality following injection of Atlantic salmon parr with the formulations shown in table 3.

Mortality in injected groups was recorded. Low to moderate mortality was observed in all groups injected with a volume of 0.05 ml, regardless of hexaflumuron concentration. Mortality increased dramatically with injection volume, reaching >90% when the injection volume was 0.2 ml. This mortality was not dependent on the presence of hexaflumuron in the formulation. The results demonstrate that formulations based on DMSO are highly toxic to fish following intraperitoneal injection.

Specifically, even when fish are injected with the smallest volume of 0.05ml DMSO, the observed level of mortality (generally about 5%) is completely unacceptable for a product that is to be used in large populations of farmed fish. Moreover, it is not possible to increase the injection volume without significantly further increasing the level of mortality.

To investigate the toxicity of the polar solvent further, formulations comprising different amounts of DMSO were prepared as detailed in Table 4. The formulations were intraperitoneally injected in a single dose into Atlantic salmon (19 - 38 g) kept in freshwater (8 - 17 °C). The eventual toxicity induced by each formulation was assessed by continuous registration of mortality up until 3 months post injection, and is shown in Table 4.

**Table 4**

| **Hexaflumuron (mg/ml)** | **DMSO (mass%)** | **Injection vol. (ml)** | **Fish mass (g)** | **Injection dose DMSO (g/kg)** | **Toxicity (% Mortality)** |
|---|---|---|---|---|---|
| 25 | 85 | 0.05 | 35.5 | 1.3 | 7 |
| 25 | 85 | 0.1 | 35.5 | 2.5 | 33 |
| 25 | 85 | 0.2 | 26.3 | 6.9 | 84 |
| 25 | 85 | 0.2 | 22.6 | 8.0 | 100 |
| 50 | 68 | 0.05 | 20 | 1.8 | 15 |
| 25 | 68 | 0.2 | 37.8 | 3.8 | 40 |
| 50 | 55 | 0.05 | 20 | 1.5 | 0 |
| 0 | 45 | 0.2 | 35.4 | 2.7 | 20 |
| 25 | 15 | 0.2 | 26.3 | 1.2 | 4 |
| 25 | 15 | 0.05 | 19 | 0.4 | 0 |
| 25 | 10 | 0.05 | 19 | 0.3 | 0 |
| 25 | 5 | 0.05 | 19 | 0.1 | 0 |

Figure 2 shows the correlation between the injected dose of DMSO (g/kg fish) and the toxicity (% mortality).

Figure 2 shows that the toxicity (% mortality) increases linearly (R² = ^{0.97}) with the injected dose of DMSO (g/kg fish). DMSO was found to be toxic to Atlantic salmon at doses above approximately 1 g/kg. The LD50 for salmonids has been reported to be between 12-17 g/kg (Benville et al, Toxicology And Applied Pharmacology 12, 156-178 (1968)) and a dose of 7.05 g/kg was reported not to give any mortality in salmonids. Therefore, it was extremely surprising to find that doses as low as about 1 g/kg cause mortality in Atlantic salmon.

In view of the surprising and unexpected toxicity observed with formulations comprising DMSO, formulations comprising other polar solvents were produced, and the safety of these formulations for intraperitoneal injection of fish was tested.

Atlantic salmon (average size: 37.8 g) kept in freshwater (17°C) were divided into 11 groups of 5 or 25 fish per group. Each group received an injection (0.2 ml) of one of the formulations shown in Table 5. The formulations were based on different solvents as indicated in table 5. Mortality was then logged over a 3 week period. The results demonstrated that all solvents tested were highly toxic to fish when injected intraperitoneally, except for the control groups, PG and PBS, which were safe (see Table 5).

**Table 5**

| **Group** | **Contents (mass%)** | **Injected fish (n)** | **Dead fish (n)** | **% Mortality** |
|---|---|---|---|---|
| 1 | DMSO/PG (85/15) | 5 | 5 | 100 |
| 2 | DMSO/EtOH (90/10) | 5 | 5 | 100 |
| 3 | Glycofurol | 5 | 5 | 100 |
| 4 | NMP | 5 | 5 | 100 |
| 5 | Ethyl acetate | 5 | 5 | 100 |
| 6 | Ethyl acetate /DMSO (25/75) | 5 | 5 | 100 |
| 7 | Ethyl acetate /DMSO (50/50) | 5 | 5 | 100 |
| 8 | Ethyl acetate /DMSO (75/25) | 5 | 4 | 80 |
| 9 | 1-methoxy-2-propanol | 5 | 5 | 100 |
| 10 | PBS | 5 | 0 | 0 |
| 11 | PG | 25 | 0 | 0 |

### Example 3

In example 2 it was demonstrated that the unacceptable toxicity induced by solution based formulations was due to the polar solvent (DMSO) in the formulations and not the content of the parasiticide. Replacing DMSO with other polar solvents did not solve the toxicity challenges (see table 5).

In view of this, to attempt to produce formulations with a reduced toxicity, formulations were produced in which the proportion of the polar solvent in the formulations was reduced, simultaneously increasing the proportion of the excipient PG. It has previously been found that PG is safe after intraperitoneal injection in Atlantic salmon (see table 5).

However, the solubility of hexaflumuron in PG has been found to be very low, as shown in table 2. As a result, it was found that it was not possible to adjust the relative proportions of PG and DMSO sufficiently to attain a formulation in which the parasiticide is soluble, and which does not induce unacceptable levels of toxicity in the fish.

This problem was surprisingly solved by additionally including in the formulations a solubilizer such as Cremophor EL (Cr. EL). Table 6 shows the composition of various formulations of parasiticide (in this case hexaflumuron) comprising DMSO, Cr. EL, and PG.

The formulations may be produced by a number of different methods and the skilled person will be familiar with suitable methods. In the present Examples, the formulations of parasiticide were prepared by dissolving parasiticide (and organic acid where indicated) under stirring at ambient temperature in a premix of polar solvent, solubilizer and/or excipient (as indicated). Alternatively, the parasiticide may be first dissolved in the polar solvent under stirring at ambient temperature, followed by sequential addition of solubilizer and/or excipient (as indicated) under stirring and ambient temperature.

The parasiticide was found to have a good solubility in all of the formulations. Thus, by this approach it was surprisingly found to be possible to prepare a 25 mg/ml solution of parasiticide with as little as 5 mass% polar solvent.

In view of this, it was investigated whether the combination of polar solvent, solubilizer, and excipient could be used to prepare formulations comprising a much higher concentration of parasiticide.

**Table 6**

| **Hexaflumuron (mg/ml)** | **DMSO (mass%)** | **Cr. EL (mass%)** | **PG (mass%)** |
|---|---|---|---|
| 50 | 68 | 20 | 12 |
| 25 | 68 | 20 | 12 |
| 50 | 55 | 35 | 10 |
| 25 | 15 | 30 | 55 |
| 25 | 15 | 35 | 50 |
| 25 | 15 | 40 | 45 |
| 25 | 15 | 45 | 40 |
| 25 | 15 | 50 | 35 |
| 25 | 10 | 35 | 55 |
| 25 | 10 | 40 | 50 |
| 25 | 10 | 45 | 45 |
| 25 | 10 | 50 | 40 |
| 25 | 5 | 40 | 55 |
| 25 | 5 | 45 | 50 |
| 25 | 5 | 50 | 45 |
| 25 | 5 | 55 | 40 |
| 25 | 5 | 60 | 35 |

Table 7 shows the composition of a number of formulations that have been successfully prepared, comprising parasiticide (in this case hexaflumuron) present in stable solution at high concentration. Using a combination of polar solvent, solubilizer, and excipient, it has been found to be possible to prepare formulations comprising a high concentration of parasiticide with less than 40 mass% DMSO.

**Table 7**

| **Hexaflumuron (mg/ml)** | **DMSO (mass%)** | **Cr. EL (mass%)** | **PG (mass%)** |
|---|---|---|---|
| 100 | 39.2 | 43.6 | 7.7 |
| 200 | 37.7 | 42.2 | 2.3 |
| 200 | 37.9 | 38.0 | 6.1 |
| 300 | 36.5 | 36.4 | 1.3 |

The production of parasiticide formulations in this way is particularly advantageous because it means that the volume of the formulation that is required to be injected into the fish can be reduced. This is advantageous in the case of large fish because it means that it is not necessary to inject a large volume of formulation. The ability to administer the formulation in a small volume is also particularly important for use with fish that are vulnerable to intraperitoneal injection, such as small, light fish.

A large-scale experiment was conducted to investigate whether the novel formulations with low content of polar solvent were completely safe for intraperitoneal injection. A total of 3000 Atlantic salmon parr (mean weight at start of experiment: 19 g) kept in freshwater (8°C) were divided into 6 groups (n=500 per group) and injected intraperitoneally (0.05 ml per fish) with formulations of parasiticide (in this case hexaflumuron) or control formulations. The control-formulations used were the commercially available vaccine ALPHA JECT micro 6 (PHARMAQ AS, Norway) with or without parasiticide, and PBS. The exact contents of each formulation are given in Table 8. Mortalities were logged throughout the study period of 3 months.

**Table 8**

| **DMSO (mass%)** | **PG (mass%)** | **Cr.EL (mass%)** | **Hexaflumuron (mg/ml)** | **Injected fish (n)** | **Dead fish** | **% Mortality** |
|---|---|---|---|---|---|---|
| 15 | 40 | 45 | 25 | 499 | 3 | 0.6 |
| 10 | 40 | 50 | 25 | 500 | 3 | 0.6 |
| 5 | 40 | 55 | 25 | 500 | 2 | 0.4 |
| ALPHA JECT micro 6 | | | 0 | 500 | 2 | 0.4 |
| ALPHA JECT micro 6 | | | 25 | 500 | 0 | 0 |
| PBS | | | 0 | 500 | 4 | 0.8 |

A very low level of mortality was observed in all groups, and the novel formulations comprising a polar solvent and a solubilizer did not cause a significant level of mortality above the background levels observed in the three control groups (see table 8).

In a separate experiment, Atlantic salmon (ca 19-35g) were injected with 0.05 ml of 25 mg/ml hexaflumuron formulated in DMSO/PG (85/15) (n=210), DMSO/Cr.EL/PG (15/45/40) (n=499), or PBS (n=500). Mortality was registered for 6 days in the group injected with DMSO/PG (85/15) and for 3 months in the two remaining groups. Observed % mortality was about 3.3% (DMSO/PG), about 0.6% (DMSO/Cr.EL/PG), and about 0.8% (PBS).

The novel parasiticide formulation comprising a polar solvent and a solubilizer can therefore be regarded as safe for intraperitoneal injection into Atlantic salmon.

### Example 4

Having demonstrated that formulations suitable for intraperitoneal injection can be prepared using DMSO as an example polar solvent, it was investigated whether suitable formulations could also be produced using other polar solvents.

Atlantic salmon (approximately 18 g) kept in 17°C freshwater were intraperitoneally injected with 0.1 ml of parasiticide (in this case, hexaflumuron) formulated as solutions using different polar solvents. In each formulation, hexaflumuron was found to be present in stable solution.

The nature of the polar solvent and the relative quantities of polar solvent, solubilizer, and excipient are given in table 9 for each formulation. PBS was injected into one group as a control. All groups (n=5 per group) were observed for one week and mortalities were registered (see table 9).

**Table 9**

| **Contents of formulation** | **Ratio (mass%)** | **Injected fish (n)** | **Dead fish (n)** |
|---|---|---|---|
| PBS | 100 | 5 | 0 |
| DMSO/Cr.EL/PG | 10/50/40 | 5 | 0 |
| DMSO/Cr.EL/PG | 40/45/15 | 5 | 0 |
| NMP/Cr EL/PG | 10/50/40 | 5 | 0 |
| Tetraglycol/Cr EL/PG | 30/50/20 | 5 | 0 |
| Acetone/Cr EL/PG | 20/50/30 | 5 | 0 |
| DMF/Cr EL/PG | 10/50/40 | 5 | 0 |
| DMF/Cr EL/PG | 40/45/15 | 5 | 0 |

The results suggested that parasiticide formulations could be produced using various different polar solvents, and that formulations can be produced using each of the different polar solvents that are safe for intraperitoneal injection into fish.

### Example 5

In example 3 it was demonstrated that hexaflumuron formulations suitable for intraperitoneal injection can be prepared using a combination of polar solvent (DMSO), solubilizer (Cr. EL), and excipient (PG). In the present experiment, it was investigated whether other parasiticides could also be formulated in this way.

Various parasiticides were formulated as 25 mg/ml solutions in polar solvent (20 or 40 mass% DMSO), solubilizer (40 mass% Cr. EL) and excipient (20 or 40 mass% PG) as detailed in Table 10. A formulation comprising hexaflumuron was prepared for comparison.

In each formulation, the parasiticide was found to be present in stable solution.

**Table 10**

| **Parasiticide** | **Parasiticide (mg/ml)** | **DMSO (mass%)** | **Cr. EL (mass%)** | **PG (mass%)** |
|---|---|---|---|---|
| Deltamethrin | 25 | 20 | 40 | 40 |
| Diflubenzuron | 25 | 40 | 40 | 20 |
| Ivermectin | 25 | 20 | 40 | 40 |
| Lufenuron | 25 | 20 | 40 | 40 |
| Emamectin | 25 | 20 | 40 | 40 |
| Hexaflumuron | 25 | 20 | 40 | 40 |

In another experiment, the feasibility of preparing a combination product comprising hexaflumuron and a second parasiticide in the same product was tested. Formulations comprising hexaflumuron (12.5 mg/ml) and one of 7 other parasiticides (each at 12.5 mg/ml) were prepared in polar solvent (20 or 30 mass% DMSO), solubilizer (40 mass% Cr. EL), and excipient (30 or 40 mass% PG) as detailed in table 11.

**Table 11**

| **First Parasiticide (12.5 mg/ml)** | **Second Parasiticide (12.5 mg/ml)** | **DMSO (mass%)** | **Cr. EL (mass%)** | **PG (mass%)** |
|---|---|---|---|---|
| Hexaflumuron | Deltamethrin | 20 | 40 | 40 |
| Hexaflumuron | Ivermectin | 20 | 40 | 40 |
| Hexaflumuron | Lufenuron | 20 | 40 | 40 |
| Hexaflumuron | Emamectin | 20 | 40 | 40 |

In each formulation, the parasiticides were found to be present in stable solution.

### Example 6

Atlantic salmon were injected with a parasiticide (hexaflumuron) formulated in 3 different formulations: a 25 mg/ml solution in a polar solvent (85 mass% DMSO and 15 mass% PG), a water-in-oil emulsion with 25 mg/ml hexaflumuron dispersed in the continuous oil phase, and as a 50 mg/ml aqueous suspension.

In the emulsion formulation, hexaflumuron was finely dispersed in the continuous oil phase by standard high shear mixing. The same method was used for the emulsification process. In the aqueous suspension, hexaflumuron was finely dispersed by standard high shear mixing, using 0.1% Tween 80 to aid in the dispersion process and 0.4% hydroxyethylcellulose to stabilize the suspension.

The injection volume was adjusted to fish size so that all fish received an injection of 25 mg/kg, except for the group injected with the aqueous suspension, which received 125 mg/kg.

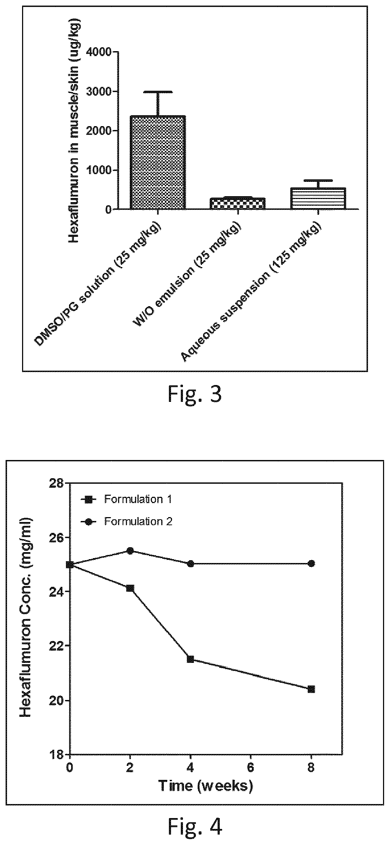

The results of the study are shown in Figure 3. The concentration of hexaflumuron was measured one week after injection in a portion of muscle and skin. Error bars indicate standard error of the mean.

The results, shown in Figure 3, surprisingly demonstrate significantly superior uptake of the polar solvent-based formulation (DMSO/PG). This unexpected result demonstrates that formulations comprising a polar solvent provide a significantly improved bioavailability of parasiticides in comparison to previously available compositions.

Atlantic salmon (average size: 25.6 g) kept in freshwater (17°C) were injected with 0.05 ml of one of the formulations shown in table 12. A portion of skeletal muscle with skin was sampled from 10 fish in each group 6 days post injection. The concentration of hexaflumuron in each tissue sample was determined.

The results of the experiment are shown in table 12. The content of each formulation is indicated and the mean concentration of hexaflumuron detected in muscle and skin 6 days post injection is shown (n=10).

**Table 12**

| **DMSO (%)** | **Cr. EL (%)** | **PG (%)** | **Hexaflumuron (mg/ml)** | **Tissue Conc. of Hexaflumuron (ug/kg)** |
|---|---|---|---|---|
| 85 | 0 | 15 | 25 | 2726 |
| 45 | 0 | 55 | 25 | 2692 |
| 45 | 17.5 | 37.5 | 25 | 3849 |
| 45 | 35 | 20 | 25 | 3510 |
| 40 | 17.5 | 42.5 | 25 | 2955 |
| 40 | 35 | 25 | 25 | 5538 |
| 30 | 35 | 35 | 25 | 5965 |

The results surprisingly and unexpectedly demonstrate that the concentration of hexaflumuron in muscle and skin was significantly increased as the relative amount of the solubilizer (Cr. EL) in the formulation was increased from 0 to 35%.

### Example 7

Having demonstrated that formulations suitable for intraperitoneal injection can be prepared using Cr. EL as a solubilizer, it was investigated whether suitable formulations could also be produced using alternative solubilizers.

In the experiment, parasiticide (in this case, hexaflumuron) was formulated using either Cr. EL or various other solubilizers.

The nature of the solubilizer and the relative quantities of polar solvent, solubilizer, and excipient are given in table 13 for each formulation.

**Table 13**

| **Hexaflumuron (mg/ml)** | **DMSO (mass%)** | **Solubilizer** | **Solubilizer (mass%)** | **Excipient** | **Excipient (mass%)** |
|---|---|---|---|---|---|
| 25 | 15 | Cr. EL | 45 | PG | 40 |
| 25 | 15 | Tween 80 | 35 | PG | 50 |
| 25 | 15 | Tween 80 | 40 | PG | 45 |
| 25 | 15 | Tween 80 | 45 | PG | 40 |
| 25 | 10 | Tween 80 | 40 | PG | 50 |
| 25 | 10 | Tween 80 | 45 | PG | 45 |
| 25 | 10 | Tween 80 | 50 | PG | 40 |
| 25 | 5 | Tween 80 | 45 | PG | 50 |
| 25 | 5 | Tween 80 | 50 | PG | 45 |
| 25 | 5 | Tween 80 | 55 | PG | 40 |
| 12,5 | 15 | Tween 20 | 45 | PG | 40 |
| 25 | 15 | Tween 20 | 45 | PG | 40 |
| 12,5 | 15 | Brij C10 | 45 | PG | 40 |
| 12,5 | 15 | Kolliphor HS15 | 45 | PG | 40 |
| 25 | 15 | Kolliphor HS15 | 45 | PG | 40 |
| 12,5 | 15 | Cr. RH40 | 45 | PG | 40 |
| 25 | 15 | Cr. RH40 | 45 | PG | 40 |

In each formulation, hexaflumuron was found to be soluble. The results suggest that suitable parasiticide formulations can be produced using different solubilizers.

### Example 8

In this experiment it was investigated whether suitable formulations could be produced using different excipients. Formulations were produced as shown in table 14.

In each formulation, the parasiticide (hexaflumuron) was found to be present in stable solution. The results suggest that suitable parasiticide formulations could be produced using different excipients.

**Table 14**

| **Hexaflumuron (mg/ml)** | **DMSO (mass%)** | **Cr. EL (mass%)** | **Excipient** | **Excipient (mass%)** |
|---|---|---|---|---|
| 25 | 15 | 45 | PG | 40 |
| 25 | 15 | 45 | Propylene carbonate | 40 |
| 25 | 15 | 45 | Ethanol | 40 |
| 25 | 15 | 45 | Butylene glycol | 40 |
| 25 | 15 | 45 | Isopropanol | 40 |
| 25 | 15 | 45 | n-butanol | 40 |

An experiment was performed in order to test if parasiticide solutions could be formulated using an excipient comprising a polar oil. Isopropyl myristate (IPM) was used as an example polar oil, and formulations were produced comprising IPM as an excipient. Table 15 shows the various formulations prepared.

**Table 15**

| **Hexaflumuron (mg/ml)** | **DMSO (mass%)** | **Cr. EL (mass%)** | **IPM (mass%)** |
|---|---|---|---|
| 25 | 15 | 45 | 40 |
| 25 | 15 | 47.5 | 37.5 |
| 25 | 15 | 50 | 35 |
| 25 | 15 | 52.5 | 32.5 |
| 50 | 15 | 52.5 | 32.5 |
| 100 | 15 | 52.5 | 32.5 |
| 25 | 15 | 55 | 30 |
| 25 | 10 | 50 | 40 |
| 25 | 10 | 52.5 | 37.5 |
| 25 | 10 | 55 | 35 |
| 25 | 10 | 57.5 | 32.5 |
| 25 | 10 | 60 | 30 |
| 25 | 5 | 60 | 35 |

All the formulations were clear solutions, in which hexaflumuron was in stable solution, indicating that polar oils such as IPM can be used as an excipient in the production of formulations comprising a parasiticide.

### Example 9

In this experiment the influence of adding a stabiliser, in this case a pH modulator, on the chemical stability of hexaflumuron in solution formulations was investigated. Citric acid was used as an example pH modulator. Formulations were produced as shown in table 16.

**Table 16**

| **Formulation** | **Hexaflumuron (mg/ml)** | **Citric Acid (volume%)** | **DMSO (mass%)** | **Cr.EL (mass%)** | **PG (mass%)** |
|---|---|---|---|---|---|
| 1 | 25 | 0 | 15 | 45 | 40 |
| 2 | 25 | 0.3 | 15 | 45 | 40 |

The formulations were incubated under stressed conditions at 40 °C and 75% relative humidity for 8 weeks. Samples for hexaflumuron analysis were withdrawn at the start of the experiment and after 2, 4 and 8 weeks. The hexaflumuron content was assayed by an HPLC method with UV detection.

The results, shown in Figure 4, demonstrate that hexaflumuron formulations comprising a stabiliser are chemically stable during the experiment. In contrast, in the absence of a stabiliser, hexaflumuron decomposes quite rapidly. This demonstrates that it may be advantageous under certain circumstances to add a stabiliser to the hexaflumuron formulations.

### Example 10

It was investigated whether suitable formulations could be produced comprising a hydrophilic polymer. The hydrophilic polymer was used to replace the excipient used in earlier Examples. Formulations were produced as shown in Table 17.

The formulations may be produced by a number of different methods and the skilled person will be familiar with suitable methods. In the present Examples, the formulations of parasiticide were prepared by dissolving parasiticide (and organic acid where indicated) under stirring at ambient temperature in a premix of polar solvent and hydrophilic polymer (as indicated). Alternatively, the parasiticide may be first dissolved in the polar solvent under stirring at ambient temperature, followed addition of hydrophilic polymer (as indicated) under stirring and ambient temperature.

**Table 17**

| **Hexaflumuron (mg/ml)** | **DMSO (mass%)** | **Cr. EL (mass%)** | **Hydrophilic Polymer** | **Hydrophilic Polymer (mass%)** |
|---|---|---|---|---|
| 25 | 15 | 50 | PEG300 | 35 |
| 25 | 15 | 50 | PEG600 | 35 |

In each formulation, the parasiticide (hexaflumuron) was found to be present in stable solution. The results suggest that suitable parasiticide formulations could be produced comprising a hydrophilic polymer.

To investigate the use of hydrophilic polymers further, an experiment was performed to investigate the solubility of a parasiticide (hexaflumuron) as a function of the molecular weight of a hydrophilic polymer, in this case, PEG. The formulation studied comprised DMSO (15 mass%), Cr. EL (50 mass%), PEG (35 mass%), and a stabiliser, citric acid (CA) (0.3 volume%).

Increasing amounts of hexaflumuron were dissolved in different versions of the formulation comprising PEG having molecular weights ranging from 200 to 700.

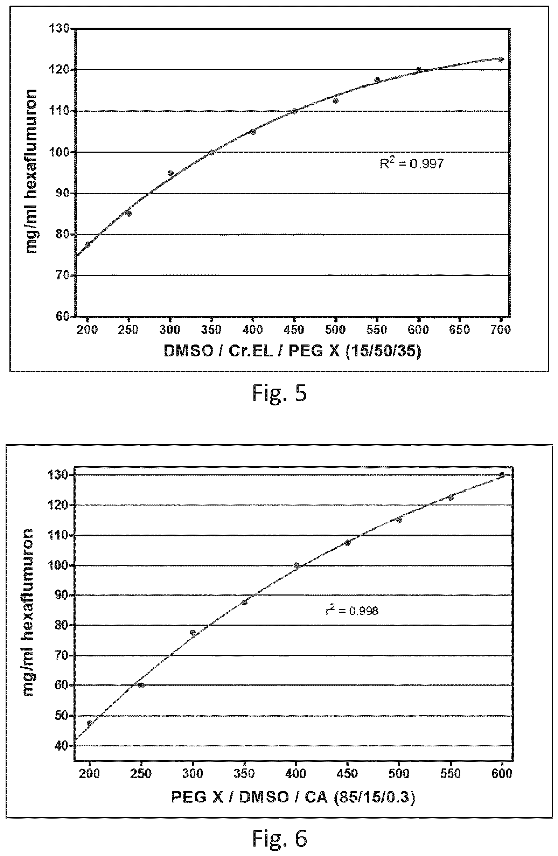

Figure 5 indicates the maximum solubility of hexaflumuron (mg/ml) as a function of PEG molecular weight.

It is evident from Figure 5 that the solubility of hexaflumuron increases significantly with increasing PEG molecular weight.

In view of this finding, an experiment was performed to investigate the solubility of parasiticide (hexaflumuron) in formulations comprising DMSO, Cr. EL, and PEG400. Formulations were produced as shown in Table 18. The formulations were prepared by dissolving increasing amounts of hexaflumuron under stirring at ambient temperature in a premix of polar solvent, solubilizer and PEG400 (as indicated) until saturation.

**Table 18**

| **Hexaflumuron (mg/ml)** | **DMSO (mass%)** | **Cr. EL (mass%)** | **PEG400 (mass%)** |
|---|---|---|---|
| 290 | 35 | 50 | 15 |
| 260 | 35 | 50 | 15 |
| 240 | 30 | 50 | 15 |
| 230 | 30 | 50 | 20 |
| 190 | 25 | 50 | 25 |
| 150 | 20 | 50 | 30 |
| 105 | 15 | 60 | 25 |
| 105 | 15 | 50 | 35 |
| 105 | 15 | 45 | 40 |
| 85 | 10 | 60 | 30 |
| 85 | 10 | 55 | 35 |
| 85 | 10 | 50 | 40 |

In each formulation, all of which were clear solutions, the parasiticide (hexaflumuron) was found to be present in stable solution. The results suggest that suitable parasiticide formulations can be produced using different ratios of solubilizer and hydrophilic polymer. Moreover, in this way, hexaflumuron formulations can be produced having an advantageously high concentration of hexaflumuron.

An experiment was then performed in order to investigate if it was possible to prepare suitable formulations using different hydrophilic polymers in combination with a polar solvent. Formulations were produced as shown in Table 19.

**Table 19**

| **Hexaflumuron (mg/ml)** | **DMSO (mass%)** | **Cr. EL (mass%)** | **Hydrophilic Polymer (Molecular Weight)** | **Hydrophilic Polymer (mass%)** |
|---|---|---|---|---|
| 100 | 15 | 50 | Poly(ethylene glycol) methyl ether (350) | 35 |
| 100 | 15 | 50 | Poly(ethylene glycol) dimethyl ether (500) | 35 |
| 100 | 15 | 50 | Poly(ethylene glycol) tetrahydrofurfuryl ether (200) | 35 |
| 50 | 15 | 50 | Trimethylolpropane ethoxylate (1014) | 35 |
| 50 | 15 | 50 | Poly(propylene glycol) (425) | 35 |

All the formulations were found to be clear homogenous solutions, in which hexaflumuron was in stable solution, demonstrating that parasiticide formulations comprising a polar solvent, a solubilizer and a hydrophilic polymer can be prepared using various different hydrophilic polymers.

In view of these findings, it was investigated whether suitable parasiticide formulations comprising hydrophilic polymers can be produced using alternative solubilizers. In the experiment, parasiticide (in this case, hexaflumuron) was formulated using a hydrophilic polymer (PEG300) and different solubilizers, as indicated in Table 20.

**Table 20**

| **Hexaflumuron (mg/ml)** | **DMSO (mass%)** | **Solubilizer** | **Solubilizer (mass%)** | **PEG300 (mass%)** |
|---|---|---|---|---|
| 12,5 | 15 | Kolliphor HS15 | 45 | 40 |
| 25 | 15 | Kolliphor HS15 | 45 | 40 |
| 50 | 15 | Kolliphor HS15 | 45 | 40 |
| 75 | 15 | Kolliphor HS15 | 45 | 40 |
| 100 | 15 | Kolliphor HS15 | 45 | 40 |
| 25 | 15 | Cr. RH40 | 45 | 40 |
| 50 | 15 | Cr. RH40 | 45 | 40 |
| 75 | 15 | Cr. RH40 | 45 | 40 |
| 100 | 15 | Cr. RH40 | 45 | 40 |

In each formulation, hexaflumuron was found to be soluble, and to form a stable homogeneous solution. The results suggest that suitable parasiticide formulations can be produced using a polar solvent, a hydrophilic polymer, and various different solubilizers. Advantageously, formulations having a broad range of parasiticide concentrations, of at least 12.5-100mg/ml, can be produced.

### Example 11

In view of the surprisingly high solubility of parasiticides such as hexaflumuron in hydrophilic polymers, it was hypothesised that it may be possible to produce formulations comprising a polar solvent and a hydrophilic polymer, without the need for a solubilizer.

To investigate this, formulations were prepared as shown in Table 21. The formulations were prepared by dissolving increasing amounts of hexaflumuron under stirring at ambient temperature in a premix of polar solvent, solubilizer and PEG400 (as indicated) until saturation.

**Table 21**

| **Hexaflumuron (mg/ml)** | **DMSO (mass%)** | **Cr.EL (mass%)** | **PEG400 (mass%)** |
|---|---|---|---|
| 90 | 15 | 55 | 45 |
| 90 | 15 | 0 | 85 |
| 140 | 20 | 0 | 80 |
| 180 | 25 | 0 | 75 |
| 200 | 30 | 0 | 70 |
| 215 | 30 | 0 | 70 |
| 260 | 35 | 0 | 65 |

All the formulations were clear solutions. Hexaflumuron was found to be soluble, and to form a stable homogeneous solution in formulations comprising a polar solvent and a hydrophilic polymer, without the need for a solubilizer. Moreover, in this way, hexaflumuron formulations can be produced having an advantageously high concentration of hexaflumuron.

An experiment was performed in order to investigate the solubility of parasiticide (hexaflumuron) in a formulation comprising PEG (85 mass%), DMSO (15 mass%), and CA (0.3 volume %) as a function of PEG molecular weight. Increasing amounts of hexaflumuron were dissolved in different mixtures of PEG/DMSO/CA (85/15/0.3) with PEG molecular weights ranging from 200 to 600. Figure 6 shows the maximum solubility of hexaflumuron (mg/ml) as a function of PEG molecular weight.

It is evident from Figure 6 that the solubility of hexaflumuron increases significantly with PEG molecular weight. In a formulation comprising 85% by weight PEG600 and 15% by weight DMSO, the maximum solubility of hexaflumuron is around 130 mg/ml.

An experiment was performed in order to investigate if it was possible to prepare similar pesticide formulations comprising a polar solvent using different hydrophilic polymers. Formulations were prepared as shown in Table 22.

**Table 22**

| **Hexaflumuron (mg/ml)** | **DMSO (mass%)** | **Hydrophilic Polymer (Molecular weight)** | **Hydrophilic Polymer (mass%)** |
|---|---|---|---|
| 100 | 15 | Poly(ethylene glycol) methyl ether (350) | 85 |
| 100 | 15 | Poly(ethylene glycol) dimethyl ether (500) | 85 |
| 100 | 15 | Poly(ethylene glycol) tetrahydrofurfuryl ether (200) | 85 |
| 50 | 15 | Trimethylolpropane ethoxylate (1014) | 85 |
| 50 | 15 | Poly(propylene glycol) (425) | 85 |

All the formulations were clear solutions, in which hexaflumuron was in stable solution, demonstrating that pesticide formulations comprising a polar solvent and a hydrophilic polymer can be prepared using various different hydrophilic polymers.

An experiment was performed to investigate the delivery of parasiticide to fish tissues by intraperitoneal injection of formulations comprising a hydrophilic polymer and a polar solvent, but no solubilizer.

Atlantic salmon of average weight 35 grams (on average 25 fish per group) were intraperitoneally injected with one of the parasiticide (hexaflumuron) formulations indicated in Table 22. The formulations also contained 0.3% citric acid. The injection volume was 0.05ml.

The fish were kept in fresh water at 12°C. The concentration of hexaflumuron was measured, in a portion of muscle and skin, 6 weeks after injection. The results of the study are shown in Table 23.

**Table 23**

| **Formulation** | **Solubilizer Present** | **Hexaflumuron concentration** |
|---|---|---|
| 1 (DMSO/Cr. EL/PEG400) | Yes | 21889 |
| 2 (DMSO/PEG400) | No | 18841 |

The results demonstrate that both formulations generate a substantial and significant concentration of parasiticide in the tissues of the treated fish six weeks after injection. Clearly, suitable parasiticide formulations can be produced using a polar solvent in combination with either one or both of a solubilizer or a hydrophilic polymer, such as PEG.

### Example 12

In view of the above findings, the potential of hydrophilic polymers such as PEG in the production of suitable parasiticide formulations was investigated further.

Formulations were produced comprising a hydrophilic polymer (PEG300), optionally together with a polar solvent and/or solubilizer, as indicated in Table 24.

**Table 24**

| **Formulation** | **Hexaflumuron (mg/ml)** | **DMSO (mass%)** | **Solubilizer (mass%)** | **Hydrophilic Polymer (mass %)** | **Excipient (mass%)** |
|---|---|---|---|---|---|
| 1 | 25 | 0 | 0 | PEG300 (100) | 0 |
| 2 | 25 | 15 | Cr EL (45) | 0 | PG (40) |
| 3 | 25 | 15 | Cr EL (50) | 0 | IPM (35) |
| 4 | 25 | 15 | Cr EL (47.5) | PEG300 (37.5) | 0 |
| 5 | 25 | 0 | Cr EL (60) | PEG300 (40) | 0 |
| 6 | 25 | 0 | Tween 80 (1) | PEG300 (99) | 0 |
| 7 | 0 | 0 | 0 | 0 | PG (100) |
| 8 | 0 | 0 | 0 | 0 | IPM (100) |
| 9 | 0 | 0 | 0 | PEG300 (100) | 0 |

In each formulation in which a parasiticide is present, the parasiticide was found to be present in a stable homogeneous solution.

It was particularly surprising that suitable formulations, comprising a parasiticide in homogeneous solution, could be produced comprising a hydrophilic polymer, but no polar solvent or solubilizer.

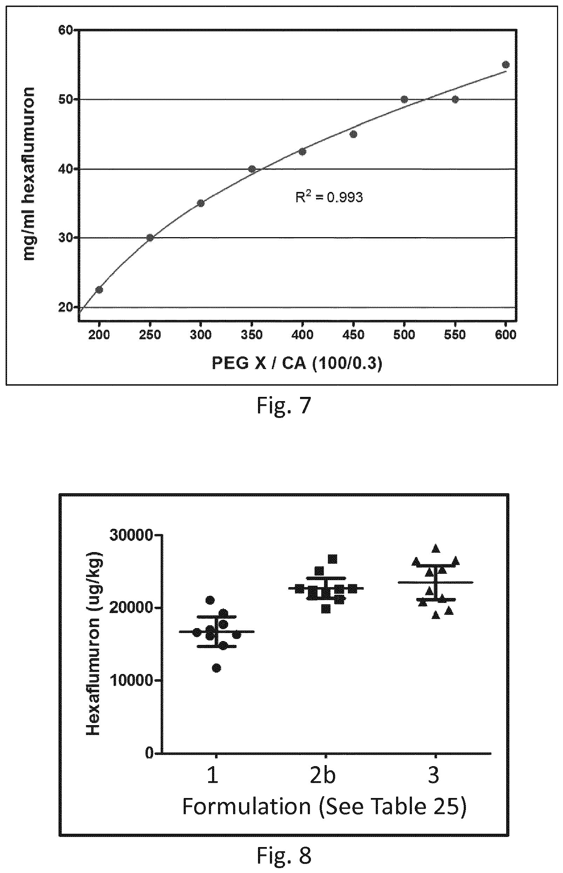

An experiment was therefore performed in order to investigate the solubility of parasiticide (hexaflumuron) in a formulation comprising PEG (100 mass%) and CA (0.3 volume %) as a function of PEG molecular weight. Increasing amounts of hexaflumuron were dissolved in different mixtures of PEG/CA (100/0.3) with PEG molecular weights ranging from 200 to 600. Figure 7 shows the maximum solubility of hexaflumuron (mg/ml) as a function of PEG molecular weight.

It is evident from Figure 7 that hexaflumuron has a significant solubility in hydrophilic polymers such as PEG, and that the solubility increases significantly with PEG molecular weight.

In view of these findings, it was investigated whether suitable formulations comprising a hydrophilic polymer could be produced with other parasiticides. Various parasiticides were formulated as 25 - 50 mg/ml solutions in PEG 400, as detailed in Table 25. The formulations were prepared by dissolving parasiticide (and organic acid where indicated) under stirring at ambient temperature in the hydrophilic polymer (as indicated).

**Table 25**

| **Parasiticide** | **Parasiticide Concentration (mg/ml)** | **PEG 400 (mass%)** |
|---|---|---|
| Deltamethtrin | 25 | 100 |
| Ivermectin | 50 | 100 |
| Lufenuron | 50 | 100 |
| Emamectin | 25 | 100 |
| Hexaflumuron | 40 | 100 |

In each formulation, the parasiticide was found to be present in stable solution.

The possibility of preparing a combination product comprising hexaflumuron and a second parasiticide was then tested. Formulations containing hexaflumuron (12.5 or 20 mg/ml) and one of 4 other parasiticides (each at 12.5 or 20 mg/ml) were prepared in PEG 400 as detailed in Table 26.

**Table 26**

| **First Parasiticide** | | **Second Parasiticide** | | **PEG 400 (mass%)** |
|---|---|---|---|---|
| **Paraciticide** | **Concentration (mg/ml)** | **Paraciticide** | **Concentration (mg/ml)** | |
| Hexaflumuron | 12,5 | Deltamethrin | 12,5 | 100 |
| Hexaflumuron | 20 | Ivermectin | 20 | 100 |
| Hexaflumuron | 20 | Lufenuron | 20 | 100 |
| Hexaflumuron | 12,5 | Emamectin | 12,5 | 100 |

In each formulation, the parasiticide was found to be present in stable solution.

In view of the finding that formulations suitable for intraperitoneal injection can be prepared using different PEG with various chain lengths, it was investigated whether suitable formulations could be prepared using alternative hydrophilic polymers. Formulations were produced as shown in Table 27.

**Table 27**

| **Hydrophilic Polymer** | **Molecular Weight** | **Hexaflumuron Concentration (mg/ml)** |
|---|---|---|
| Poly(propylene glycol) | 425 | 10 |
| Poly(propylene glycol) | 425 | 20 |
| Methoxy poly(ethylene glycol) | 350 | 10 |
| Methoxy poly(ethylene glycol) | 350 | 80 |
| Methoxy poly(ethylene glycol) | 550 | 10 |
| Methoxy poly(ethylene glycol) | 550 | 70 |
| Poly(ethylene glycol) methyl ether | 350 | 80 |
| Poly(ethylene glycol) methyl ether | 550 | 75 |
| Poly(ethylene glycol) tetrahydrofurfuryl ether | 200 | 80 |
| Poly(ethylene glycol) tetrahydrofurfuryl ether | 200 | 100 |
| Glycerol propoxylate | 1500 | 10 |
| Poly(ethylene glycol) dimethyl ether | 250 | 80 |
| Poly(ethylene glycol) dimethyl ether | 250 | 140 |
| Poly(ethylene glycol) dimethyl ether | 500 | 80 |
| Poly(ethylene glycol) dimethyl ether | 500 | 10 |
| Poly(ethylene glycol) diglycidyl ether | 500 | 30 |
| Poly(ethylene glycol) diglycidyl ether | 500 | 42 |
| Trimethylolpropane ethoxylate | 170 | 24 |
| Trimethylolpropane ethoxylate | 450 | 12 |
| Poly(propylene glycol) | 425 | 20 |
| Poly(propylene glycol) | 725 | 10 |
| Poly(propylene glycol) monobutyl ether | 340 | 10 |
| Poly(propylene glycol) diglycidyl ether | 380 | 20 |

In each formulation, the parasiticide was found to be present in stable solution. The results suggest that suitable parasiticide formulations can be produced using different hydrophilic polymers, each with a molecular weight of less than 2000. Moreover, in this way, formulations can be produced having a concentration of hexaflumuron.

### Example 13

An experiment was performed to investigate the toxicity of formulations comprising a hydrophilic polymer. Atlantic salmon having an average size of 28.7g were kept in freshwater (17°C) in 500L tanks. Each group received an intraperitoneal injection (0.2 ml) of one of the formulations shown in Table 24. A stabiliser (CA) was included in the formulation in an amount of 0.3 volume%. Fish behaviour and mortalities were monitored each day post injection, and the results are shown in Table 28.

**Table 28**

| **Formulation** | **Contents** | **Injection vol (ml)** | **Injected fish** | **Dead fish (n)** |
|---|---|---|---|---|
| 1 | PEG300 | 0.2 | 10 | 0 |
| 2a | DMSO/Cr.EL/PG/CA | 0.2 | 20 | 1 |
| 2b | DMSO/Cr.EL/PG/CA | 0.05 | 10 | 0 |
| 3 | DMSO/Cr.EL/IPM/CA | 0.05 | 10 | 0 |
| 4 | DMSO/Cr.EL/PEG300/CA | 0.2 | 20 | 1 |
| 5 | Cr.EL/PEG300/CA | 0.2 | 20 | 0 |
| 6 | Tween 80/PEG300/CA | 0.2 | 20 | 2 |
| 7 | IPM | 0.2 | 10 | 0 |
| 8 | PG | 0.2 | 10 | 0 |
| 9 | PBS | 0.2 | 20 | 0 |

The results clearly show that the safety profile of formulations comprising PEG300 was similar to that of DMSO/Cr. EL formulations. In particular, it was surprisingly and advantageously found that formulations comprising a parasiticide in homogeneous solution, comprising a hydrophilic polymer, but no polar solvent or solubilizer, were not toxic to fish when injected intraperitoneally. This was the case even when the formulations were injected in large volumes (0.2ml).

The effect of formulations comprising a hydrophilic polymer on the delivery of parasiticide was tested. The concentration of hexaflumuron in skin was measured one week after intraperitoneal injection with formulation 1, 2b, or 3 (Table 28). The results are shown in Figure 8.

The results show that all of the formulations tested resulted in the accumulation of significant levels of hexaflumuron in fish tissues one week after injection. This result, in combination with the finding that the formulations are not toxic, indicates that the formulations comprising parasiticide in solution in a hydrophilic polymer, in the absence of a polar solvent, or a solubilizer, are suitable for use in delivering parasiticides to fish by intraperitoneal injection.

It was unexpectedly observed that following injection, precipitated hexaflumuron was visible in the visceral cavity. Experiments were therefore performed to investigate this observation in more detail.

### Example 14

Atlantic salmon (mean weight: 30.0 g) kept in freshwater (12°C) were divided into 6 groups of 30 fish per group according to Table 29. Each group received an intraperitoneal injection (0.05 ml) of a formulation as indicated in Table 29. Hexaflumuron formulated in a water/oil emulsion was used as a control group (Formulation 6).

**Table 29**

| **Formulation** | **Hexaflumuron (mg/ml)** | **DMSO (mass%)** | **Cr EL (mass%)** | **PG (mass%)** | **PEG300 (mass%)** |
|---|---|---|---|---|---|
| 1 | 25 | 15 | 45 | 40 | 0 |
| 2 | 25 | 15 | 50 | 0 | 35 |
| 3 | 90 | 15 | 50 | 0 | 35 |
| 4 | 25 | 0 | 0 | 0 | 100 |
| 5 | 25 | 0 | 0 | 0 | 100 |
| 6 | 25 | 0 | 0 | 0 | 0 |

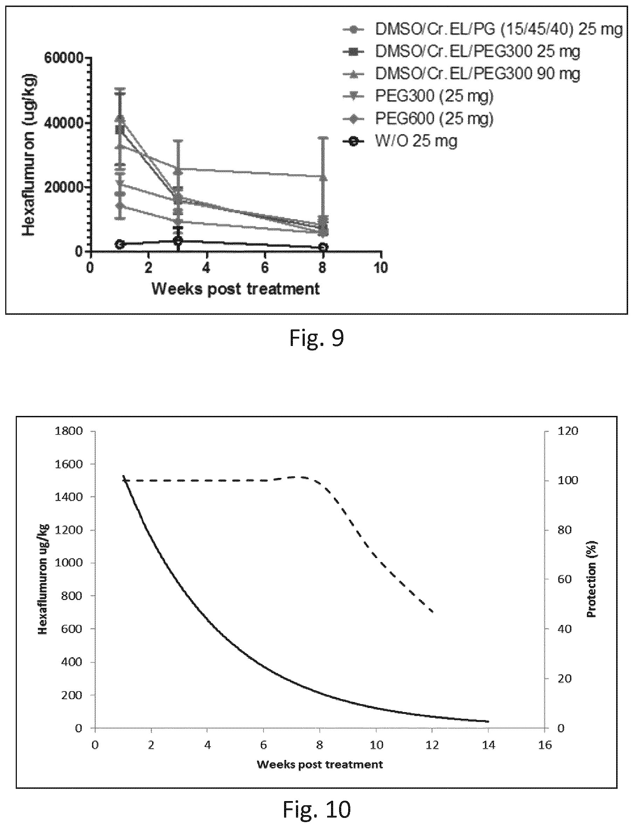

No mortalities or obvious changes in behavior were observed as a result of any of the injections. The skin of a full fillet from 10 fish from each group was sampled 1, 3 and 8 weeks post injection and the concentration of hexaflumuron was measured. The results are shown in Figure 9. Error bars represent 95% confidence intervals with a line indicating the mean.

It is clear from Figure 9 that the mean hexaflumuron tissue concentration at each time-point differed considerably between groups. Two groups, Formulations 1 and 2, showed very high tissue concentration one week after injection, followed by rapid depletion. The other groups had lower peak measurement one week after injection, but slower depletion (Figure 9).

The intraperitoneal cavity was inspected for local reactions one and three weeks after injection. A white precipitate on the internal organs was consistently observed in some of the groups (see Table 30).

**Table 30**

| **Formulation** | **Hexaflumuron (mg/ml)** | **White residues/precipitate in peritoneal cavity** | |
|---|---|---|---|
| | | Week 1 | Week 3 |
| 1 | 25 | No | No |
| 2 | 25 | No | No |
| 3 | 90 | Yes | Yes |
| 4 | 25 | Yes | Yes |
| 5 | 25 | Yes | Yes |
| 6 | 25 | No | (Yes) * |

| | | | |
|---|---|---|---|
| *Probably not precipitated Hexaflumuron, but oil-drops | | | |

The depletion kinetics in Figure 9 suggested that the half-life of Hexaflumuron in the fish tissues strongly depended upon the formulation. Formulations that depleted more slowly also formed a white precipitate in the peritoneal cavity (Table 30), indicating that this precipitate could function as a depot.

Table 31 shows the tissue concentration of hexaflumuron from weeks 1 to 8. From this information, the half-life of Hexaflumuron in skin was calculated for each formulation. The estimated mean half-lives varied from a minimum of 17.35 days for Formulation 1 to a maximum of 95.57 days for Formulation 3. The theoretical duration of efficacy (assuming a threshold of efficacy of approximately 100µg/kg) was estimated directly for each formulation using the representative half-lives. A conservative estimate was also made assuming a minimum half-life of 17.35 days after week 8. This was done since it can be expected that the depot effect will be reduced after some time. Based on this analysis, Formulation 3 is estimated to be efficient for at least 179 days, and for a maximum of approximately 765 days at 120°C when 30g fish are injected with a 0.05 ml dose.

**Table 31**

| **Form.** | **Week 1 (µg/kg)** | **Week 8** | | **Half-life (Days)** | **Remaining Half-lives (Days)** | **Duration of Efficacy (Days)** | |
|---|---|---|---|---|---|---|---|
| | | **(µg/kg)** | **%** | | | **Max** | **Min** |
| 1 | 41420 | 5848 | 14 | 17.35 | 5.5 | 144.425 | 144.425 |
| 2 | 37930 | 7285 | 19 | 20.59 | 6 | 172.54 | 153.1 |
| 3 | 33060 | 23172 | 70 | 95.57 | 7.5 | 765.775 | 179.125 |
| 4 | 20810 | 8310 | 40 | 37 | 6.5 | 289.5 | 161.775 |
| 5 | 14197 | 5769 | 41 | 37.72 | 5.5 | 256.46 | 144.425 |
| 6 | 2331 | 1317 | 56 | 59.49 | 3.5 | 257.215 | 68.25 |

When depletion kinetics are compared for different injection formulations up to 8 weeks after injection, significant differences are observed. Formulations that that provide the greatest tissue concentrations one week after injection do not necessarily provide the longest duration of protection.

In particular, formulations that do not comprise a solubilizer have been found upon intraperitoneal injection to result in the precipitation of hexaflumuron in the peritoneal cavity. These formulations have also been found to provide a depot effect. The provision of a long-lasting duration of efficacy due to relatively high tissue levels and slow depletion, as seen in the present formulation, is a major advantage as it reduces the frequency with which parasiticides must be administered to fish. Indeed, in some cases, the half-lives may be sufficient for one 0.05ml administration to protect a fish for the entire marine growth-period. Moreover, it is also advantageous that the depot effect can easily be easily controlled by adjusting the composition of the injectable formulations.

### Example 15

Experiments were performed to investigate the relationship between the tissue concentration of parasiticide and the effect on lice numbers.

Atlantic salmon (total n=180) were kept in full seawater (3.5% salinity, 12°C). Half of the population (n=90) were treated with parasiticide (in this case hexaflumuron) on day 0. Treated and untreated fish (n=10 per group) were randomly collected from each group and challenged with copepodids of *Lepeophtheirus salmonis* in a common challenge tank during a 12 weeks period. This was done six times after treatments, in weeks 1, 3, 6, 8, 10 and 12 post treatment. The number of lice reaching chalimus stage was registered on each fish 2 weeks post challenge. Concentration of hexaflumuron in a portion of muscle with skin was also measured in each fish at the time of lice count.

Table 32 shows the mean number of lice (chalimus stage) in treated and untreated groups at different time-points after treatment.

**Table 32**

| **Weeks post treatment** | **Mean number of lice per fish** | | **Relative effect of treatment** |
|---|---|---|---|
| | **Treated group** | **Untreated group** | |
| 1 | 0 | 23.7 | 100 % |
| 3 | 0 | 8.8 | 100 % |
| 6 | 0 | 0.9 | 100 % |
| 8 | 0.2 | 16.3 | 98 % |
| 10 | 1.7 | 5.5 | 69 % |
| 12 | 27.6 | 52.0 | 47 % |

The effect of treatment at each time-point was compared with the mean concentration of hexaflumuron in the fish tissues at that time-point. Figure 10 shows a comparison of efficacy against development of chalimus stage sea lice and mean tissue levels (in a portion of muscle and skin) of hexaflumuron at indicated time-points after treatment. Efficacy is given in percentage (dotted line) and tissue concentrations in ug/kg (full line). Trend lines based on the measured data are shown.

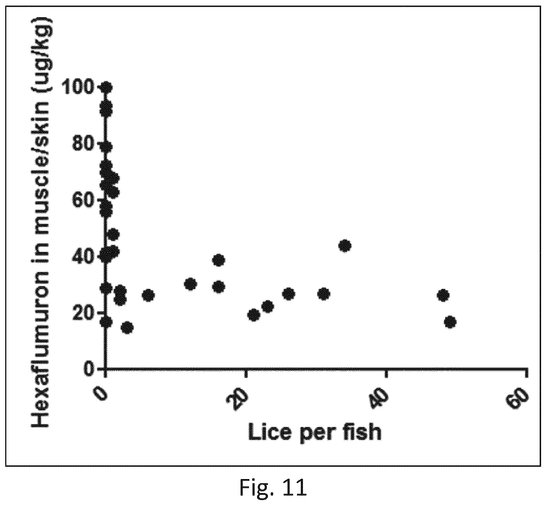

Lice numbers of each individual fish was also correlated with concentration of hexaflumuron measured in the same fish. Figure 11 shows a clear correlation between tissue concentration of hexaflumuron and lice numbers on individual fish throughout the experiment. Tissue-concentrations above 100 ug/kg are omitted for the sake of presentation. Correlation for the complete dataset is statistically significant (p<0.0001).

These analyses demonstrate a very strong correlation between tissue concentration of hexaflumuron and number of sea lice (p<0.0001). Thus the tissue concentration of hexaflumuron can be taken as a strong indicator of the potential efficacy of a given formulation in the treatment of fish against parasites.

## Claims

1. A parasiticide formulation for use in the treatment of parasitic infections of fish by intraperitoneal injection, wherein the parasiticide is hexaflumuron and wherein the formulation is not toxic to fish when administered by intraperitoneal injection, wherein the formulation is a homogeneous non-aqueous parasiticide solution, comprising:
(a) a hydrophilic polymer; or
(b) a hydrophilic polymer and a non-aqueous polar solvent, present in an amount of 5-40% by weight.

2. A formulation ; for use as claimed in claim 1, wherein the parasiticide is present in the formulation in an amount of 90-100 mg/ml, and wherein the hydrophilic polymer is present in an amount of 70-90% by weight.

3. A formulation ; for use as claimed in any of the preceding claims, wherein the hydrophilic polymer comprises poly(ethylene glycol) or poly(propylene) glycol, or a derivative thereof.

4. A formulation ; for use as claimed in claim 3, wherein the hydrophilic polymer comprises PEG300, PEG400, or PEG600.

5. A formulation ; for use as claimed in any of the preceding claims, wherein the non-aqueous polar solvent comprises one or a combination of DMSO, NMP, Tetraglycol, acetone, or DMF.

6. A formulation ; for use as claimed in any of the preceding claims, further comprising a stabiliser which is a pH control agent.

7. A formulation for use as claimed in claim 6, wherein the pH control agent comprises citric acid.

8. A parasiticide formulation for use in the treatment of parasitic infections of fish by intraperitoneal injection, wherein the formulation is not toxic to fish when administered by intraperitoneal injection, wherein the formulation is a homogeneous non-aqueous parasiticide solution, and wherein the formulation comprises:
(a) a hydrophilic polymer in an amount of about 80-90% by weight;
(b) a non-aqueous polar solvent in an amount of about 12-18% by weight;
(c) a stabilizer in an amount of about 0.2-0.4%; and,
(d) hexaflumuron in an amount of about 85-100mg/ml.

9. A formulation for use as claimed in claim 8, wherein:
(a) the hydrophilic polymer comprises PEG400 in an amount of about 85% by weight;
(b) the non-aqueous polar solvent comprises DMSO in an amount of about 15% by weight;
(c) the stabilizer comprises citric acid; and,
(d) hexaflumuron in an amount of about 90mg/ml.

10. A formulation ; for use as claimed in any of the preceding claims, wherein the formulation is suitable for treating sea lice by intraperitoneal injection of host fish.

11. A formulation for use as claimed in any of the preceding claims, wherein a therapeutically effective concentration of the parasiticide is detectable in the tissues of the fish for at least 60 days after intraperitoneal injection of the formulation.

12. A formulation for use as defined in any of claims 1 to 11 for use as an antiparasitic treatment of fish against sea lice.

13. A parasiticide formulation for use in the treatment of parasitic infections of fish by intraperitoneal injection, wherein the parasiticide is hexaflumuron and wherein the formulation is not toxic to fish when administered by intraperitoneal injection, wherein the formulation is a homogeneous non-aqueous parasiticide solution, and wherein the formulation comprises:
(a) a hydrophilic polymer;
(b) a non-aqueous polar solvent, present in an amount of 5-40% by weight; and
(c) a solubilizer.

## Patentansprüche

1. Parasitizidformulierung zur Verwendung bei der Behandlung von parasitischen Infektionen von Fischen durch intraperitoneale Injektion, wobei das Parasitizid Hexaflumuron ist und wobei die Formulierung nichttoxisch für Fische ist, wenn sie über intraperitoneale Injektion verabreicht wird, wobei die Formulierung eine homogene nichtwässrige Parasitizidlösung ist, umfassend:
(a) ein hydrophiles Polymer; oder
(b) ein hydrophiles Polymer und ein nichtwässriges polares Lösungsmittel, anwesend in einer Menge von 5-40 Gew.-%.

2. Formulierung zur Verwendung, wie in Anspruch 1 beansprucht, wobei das Parasitizid in der Formulierung in einer Menge von 90-100 mg/ml anwesend ist und wobei das hydrophile Polymer in einer Menge von 70-90 Gew.-% anwesend ist.

3. Formulierung zur Verwendung, wie in einem der vorherigen Ansprüche beansprucht, wobei das hydrophile Polymer Poly(ethylenglycol) oder Poly(propylen)glycol oder ein Derivat davon umfasst.

4. Formulierung zur Verwendung, wie in Anspruch 3 beansprucht, wobei das hydrophile Polymer PEG300, PEG400 oder PEG600 umfasst.

5. Formulierung zur Verwendung, wie in einem der vorherigen Ansprüche beansprucht, wobei das nichtwässrige polare Lösungsmittel eines oder eine Kombination von DMSO, NMP, Tetraglycol, Aceton oder DMF umfasst.

6. Formulierung zur Verwendung, wie in einem der vorherigen Ansprüche beansprucht, zusätzlich umfassend einen Stabilisierer, welcher ein pH-Kontrollmittel ist.

7. Formulierung zur Verwendung, wie in Anspruch 6 beansprucht, wobei das pH-Kontrollmittel Citronensäure umfasst.

8. Parasitizidformulierung zur Verwendung bei der Behandlung von parasitischen Infektionen bei Fischen durch intraperitoneale Injektion, wobei die Formulierung nichttoxisch für Fische ist, wenn sie über intraperitoneale Injektion verabreicht wird, wobei die Formulierung eine homogene nichtwässrige Parasitizidlösung ist und wobei die Formulierung umfasst:
(a) ein hydrophiles Polymer in einer Menge von ungefähr 80-90 Gew.-%;
(b) ein nichtwässriges polares Lösungsmittel in einer Menge von ungefähr 12-18 Gew.-%;
(c) einen Stabilisierer in einer Menge von ungefähr 0,2-0,4% und
(d) Hexaflumuron in einer Menge von ungefähr 85-100 mg/ml.

9. Formulierung zur Verwendung, wie in Anspruch 8 beansprucht, wobei:
(a) das hydrophile Polymer PEG400 in einer Menge von ungefähr 85 Gew.-% umfasst;
(b) das nichtwässrige polare Lösungsmittel DMSO in einer Menge von ungefähr 15 Gew.-% umfasst;
(c) der Stabilisierer Citronensäure umfasst und
(d) Hexaflumuron in einer Menge von ungefähr 90 mg/ml.

10. Formulierung zur Verwendung, wie in einem der vorherigen Ansprüche beansprucht, wobei die Formulierung geeignet ist für die Behandlung von Seeläusen durch intraperitoneale Injektion des Wirtsfisches.

11. Formulierung zur Verwendung, wie in einem der vorherigen Ansprüche beansprucht, wobei eine therapeutisch wirksame Konzentration des Parasitizids in den Geweben der Fische für mindestens 60 Tage nach der intraperitonealen Injektion der Formulierung nachweisbar ist.

12. Formulierung zur Verwendung, wie in einem der Ansprüche 1 bis 11 definiert, zur Verwendung bei der antiparasitischen Behandlung von Fischen gegen Seeläuse.

13. Parasitizidformulierung zur Verwendung bei der Behandlung von parasitischen Infektionen von Fischen durch intraperitoneale Injektion, wobei das Parasitizid Hexaflumuron ist und wobei die Formulierung nichttoxisch für Fische ist, wenn sie über intraperitoneale Injektion verabreicht wird, wobei die Formulierung eine homogene nichtwässrige Parasitizidlösung ist und wobei die Formulierung umfasst:
(a) ein hydrophiles Polymer;
(b) ein nichtwässriges polares Lösungsmittel, anwesend in einer Menge von 5-40 Gew.-%; und
(c) ein Solubilisierungsmittel.

## Revendications

1. Formulation parasiticide pour une utilisation dans le traitement d'infections parasitaires chez les poissons par injection intrapéritonéale, dans laquelle le parasiticide est l'hexaflumuron et dans laquelle la formulation n'est pas toxique pour les poissons lorsqu'elle est administrée par injection intrapéritonéale, dans laquelle la formulation est une solution parasiticide non aqueuse homogène, comprenant :
(a) un polymère hydrophile ; ou
(b) un polymère hydrophile et un solvant polaire non aqueux, présent en une quantité de 5 à 40 % en poids.

2. Formulation pour une utilisation selon la revendication 1, dans laquelle le parasiticide est présent dans la formulation en une quantité de 90 à 100 mg/ml, et dans laquelle le polymère hydrophile est présent en une quantité de 70 à 90 % en poids.

3. Formulation pour une utilisation selon l'une des revendications précédentes, dans laquelle le polymère hydrophile comprend du poly(éthylène glycol) ou du poly(propylène)glycol, ou un dérivé de ceux-ci.

4. Formulation pour une utilisation selon la revendication 3, dans laquelle le polymère hydrophile comprend du PEG300, du PEG400, ou du PEG600.

5. Formulation pour une utilisation selon l'une des revendications précédentes, dans laquelle le solvant polaire non aqueux comprend du DMSO, du NMP, du tétraglycol, de l'acétone, ou du DMF, ou une combinaison de ceux-ci.

6. Formulation pour une utilisation selon l'une des revendications précédentes, comprenant en outre un stabilisant qui est un agent de régulation du pH.

7. Formulation pour une utilisation selon la revendication 6, dans laquelle l'agent de régulation du pH comprend de l'acide citrique.

8. Formulation parasiticide pour une utilisation dans le traitement d'infections parasitaires chez les poissons par injection intrapéritonéale, dans laquelle la formulation n'est pas toxique pour les poissons lorsqu'elle est administrée par injection intrapéritonéale, dans laquelle la formulation est une solution parasiticide non aqueuse homogène, et dans laquelle la formulation comprend :
(a) un polymère hydrophile en une quantité d'environ 80 à 90 % en poids ;
(b) un solvant polaire non aqueux en une quantité d'environ 12 à 18 % en poids ;
(c) un stabilisant en une quantité d'environ 0,2 à 0,4 % en poids ; et
(d) de l'hexaflumuron en une quantité d'environ 85 à 100 mg/ml.

9. Formulation pour une utilisation selon la revendication 8, dans laquelle :
(a) le polymère hydrophile comprend du PEG400 en une quantité d'environ 85 % en poids ;
(b) le solvant polaire non aqueux comprend du DMSO en une quantité d'environ 15 % en poids ;
(c) le stabilisant comprend de l'acide citrique ; et,
(d) de l'hexaflumuron en une quantité d'environ 90 mg/ml.

10. Formulation pour une utilisation sur l'une des revendications précédentes, dans laquelle la formulation est adaptée pour le traitement des poux de mer par injection intrapéritonéale à un poisson hôte.

11. Formulation pour une utilisation sur l'une des revendications précédentes, dans laquelle une concentration thérapeutiquement efficace du parasiticide est détectable dans les tissus du poisson pendant au moins 60 jours après l'injection péritonéale de la formulation.

12. Formulation pour une utilisation selon l'une des revendications 1 à 11 pour une utilisation en tant que traitement antiparasitaire des poissons contre les poux de mer.

13. Formulation parasiticide pour une utilisation dans le traitement d'infections parasitaires chez les poissons par injection intrapéritonéale, dans laquelle le parasiticide est l'hexaflumuron et dans laquelle la formulation n'est pas toxique pour les poissons lorsqu'elle est administrée par injection intrapéritonéale, dans laquelle la formulation est une solution parasiticide non aqueuse homogène, et dans laquelle la formulation comprend :
(a) un polymère hydrophile ;
(b) un solvant polaire non aqueux, présent en une quantité de 5 à 40 % en poids ; et
(c) un agent solubilisant.
